(19) Europäisches Patentamt
European Patent Office
Office européen des brevets

(11) Veröffentlichungsnummer: **0 149 088**
**B1**

(12) # EUROPÄISCHE PATENTSCHRIFT

(45) Veröffentlichungstag der Patentschrift: **18.01.89**

(21) Anmeldenummer: **84114607.9**

(22) Anmeldetag: **01.12.84**

(51) Int. Cl.⁴: **C 07 D 401/12**, C 07 D 453/02, C 07 D 451/04, C 07 D 405/14, A 61 K 31/44, A 61 K 31/445 // (C07D401/12, 211:46, 213:64),(C07D401/12, 211:22, 213:64),(C07D401/12, 211:24, 213:64)

(54) **Neue Pyridin-2-ether beziehungsweise Pyridin-2-thioether mit einem stickstoffhaltigen cycloaliphatischen Ring sowie deren Sulforide, Sulfone und Pyridin-N-oxide.**

(30) Priorität: **28.12.83 DE 3347276**

(43) Veröffentlichungstag der Anmeldung: **24.07.85 Patentblatt 85/30**

(45) Bekanntmachung des Hinweises auf die Patenterteilung: **18.01.89 Patentblatt 89/3**

(84) Benannte Vertragsstaaten: **AT BE CH DE FR GB IT LI LU NL SE**

(73) Patentinhaber: **Degussa Aktiengesellschaft, Weissfrauenstrasse 9, D-6000 Frankfurt am Main 1 (DE)**

(72) Erfinder: **Scheffler, Gerhard, Dr., Frankenwaldstrasse 19, D-6450 Hanau 6 (DE)**
Erfinder: **Engel, Jürgen, Dr., Cranachstrasse 25, D-8755 Alzenau (DE)**
Erfinder: **Jakovlev, Vladimir, Dr., Herm.Lönsstrasse 7, D-6457 Maintal 1 (DE)**
Erfinder: **Nickel, Bernd, Dr., Woogstrasse 19, D-6109 Mühltal 1 (DE)**
Erfinder: **Thiemer, Klaus, Dr., Fürstenbergstrasse 12, D-6450 Hanau 9 (DE)**

(56) Entgegenhaltungen:
**EP-A- 0 053 744**
**DE-A- 1 620 275**
**DE-A- 2 905 876**
**DE-A- 3 016 415**
**US-A- 4 288 442**

**CHEMICAL ABSTRACTS, Vol. 94, No. 23, 8. Juni 1981, Columbus, Ohio, USA ORUDZHEVA I.M.; EFENDIEV T.E.; ALIEV S.M.; "Synthesis of some derivatives of 2-mercaptopyridine" Seite 635, Spalte 2, Zusammenfassung-Nr. 192 081n**
**CHEMICAL ABSTRACTS, Vol. 98, No. 16, 18. April 1983, Columbus, Ohio, USA ORUDZHEVA I.M.; ALIEV S.M.; EFENDIEV T.E.; DZHAFAROV Z.I. "Study of some amino derivatives of 2-mercaptopyridine as inhibitors of hydrogen saturation" Seite 244, Spalte 1,**

(56) Entgegenhaltungen: (Fortsetzung)
**Zusammenfassung-Nr. 130 275c**
**CHEMICAL ABSTRACTS, Vol. 96, No. 5, 1. Februar 1982, Columbus, Ohio, USA YOSHITOMI PHARMACEUTICAL INDUSTRIES, LTD "Pyridine derivatives" Seite 652, Spalte 2, Zusammenfassung-Nr. 35 096v**
**CHEMICAL ABSTRACTS, Vol. 98, No. 9, 28, Februar 1983, Columbus, Ohio, USA SUHR, ROBERT G; MIESEL, JOHN L; "1-Benzoyl-3-(aryloxy- or arylthiopyridinyl) ureas compounds" Seite 616, Spalten 1,2; Zusammenfassung-nr. 71 944v**

**Die Akte enthält technische Angaben, die nach dem Eingang der Anmeldung eingereicht wurden und die nicht in dieser Patentschrift enthalten sind.**

EP 0 149 088 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Durch die belgische Patentschrift 630 125 sind Pyridinderivate der folgenden allgemeinen Formel

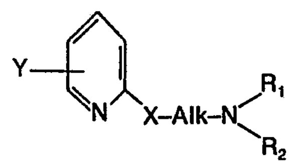

und deren Salze bekannt. In dieser Formel bedeuten $R_1$ und $R_2$ Alkylreste, vorzugsweise solche, die zu einem Ring geschlossen sind, der ein weiteres Heteroatom, insbesondere Sauerstoff, enthalten kann. Alk bedeutet eine gerade oder verzweigte niedere Alkylenkette mit höchstens 4 Kohlenstoffatomen, X bedeutet Schwefel, Sauerstoff oder die NH-Gruppe. Y kann ein Halogen, vorzugsweise in 3-Stellung, eine Alkyl-, Trihalogenmethyl- oder Alkoxygruppe, oder aber den Rest –CN, –COOR oder $-CONR_3R_4$ bedeuten, wobei R, $R_3$ und $R_4$ gleich oder verschieden sind und Wasserstoff oder niedere Alkylgruppen darstellen.

Durch die belgische Patentschrift 650 361 sind auch die entsprechenden Sulfone und Sulfoxide der zuvor genannten Verbindungen bekannt (X in der zuvor angegebenen Formel = SO oder $SO_2$).

Für diese Verbindungen wird eine analgetische beziehungsweise antiphlogistische Wirkung angegeben.

Weiterhin sind durch die europäische Patentanmeldung 21 973 4-Amino-1-(2-pyridyl)-piperidine der folgenden Formel und deren pharmazeutisch akzeptable Salze bekannt

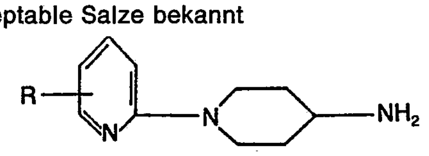

worin R für Wasserstoff, Halogen, Methyl, Trifluormethyl, Niedrigalkoxy, Trifluormethoxy, 2,2,2-Trifluoräthoxy, Niedrigalkylthio, Trifluormethylthio, Phenoxy, eine im Phenylkern durch Halogen, Trifluormethyl, Niedrigalkyl, Niedrigalkoxy, Niedrigalkylthio oder Cyano substituierte Phenoxygruppe, Phenylthio oder eine im Phenylkern durch Halogen, Trifluormethyl, Niedrigalkyl, Niedrigalkoxy, Niedrigalkylthio oder Cyano substituierte Phenylthiogruppe steht.

Für diese Verbindungen wird eine appetitzügelnde Wirkung angegeben.

Aus der US-A-4 288 442 sind
4-(2-Methyl-6-pyridyloxymethyl)piperidin und
4-(2-Pyridyloxymethyl)-piperidin
als Ausgangsmaterialien für adrenergische β-Rezeptor Hemmer bekannt.

Schliesslich werden in der deutschen Offenlegungsschrift 2 230 392 Pyridinverbindungen der folgenden allgemeinen Formel beschrieben

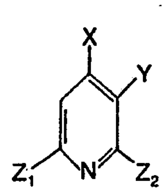

in der

X eine gegebenenfalls verzweigte und/oder substituierte Alkyl-, gegebenenfalls verzweigte und/oder substituierte Alkenyl-, gegebenenfalls substituierte Cycloalkyl-, Aralkyl-, Arylgruppe oder einen heterocyclischen Rest, oder falls Y ungleich Wasserstoff ist, auch Wasserstoff,

Y eine Cyan-, Amino-, Nitroso-, Nitro-, eine gegebenenfalls verzweigte und/oder substituierte Alkyl-, gegebenenfalls verzweigte und/oder substituierte Alkenyl-, gegebenenfalls substituierte Cycloalkyl-, Aralkylgruppe oder Reste

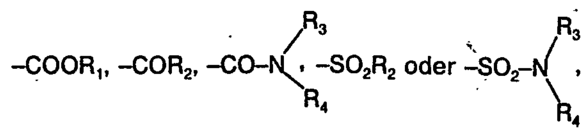

oder falls X ungleich Wasserstoff ist, auch Wasserstoff,

$Z_1$ unter anderem die Reste $-OR_{12}$, $SR_{12}$ oder $-SO_2R_{12}$

$Z_2$ unter anderem ein Chlor- oder Bromatom, eine Cyan-, Hydroxy- oder Mercaptogruppe bedeuten und

$R_1$ und $R_2$ eine gegebenenfalls verzweigte und/oder substituierte Alkyl-, eine gegebenenfalls verzweigte und/oder substituierte Alkenylgruppe, $R_2$ ferner auch eine gegebenenfalls substituierte Cycloalkyl-, Aralkyl-, Aryl- oder heterocyclische Gruppe sein kann,

$R_3$ und $R_4$ für Wasserstoff, eine gegebenenfalls verzweigte und/oder substituierte Alkyl-, gegebenenfalls substituierte Cycloalkyl-, Aralkyl- oder Arylgruppe, wobei die Alkylreste $R_3$ und $R_4$ auch direkt oder über ein Heteroatom verbunden sein können und

$R_{12}$ für eine gegebenenfalls verzweigte und/oder substituierte Alkyl-, eine gegebenenfalls verzweigte und/oder substituierte Alkenyl-, eine gegebenenfalls substituierte Cycloalkyl-, Aralkyl- oder Arylgruppe steht.

Diese Verbindungen sind Zwischenprodukte, insbesondere zur Herstellung von Farbstoffen. Es wird ausserdem angegeben, dass diese Verbindungen von Interesse seien als Schädlingsbekämpfungsmittel und für pharmazeutische Zwekke.

Die Erfindung betrifft die durch die Patentansprüche definierten Gegenstände.

Die erfindungsgemässen Verbindungen sind pharmakologisch wirksam. Insbesondere besitzen die erfindungsgemässen Verbindungen eine ausgeprägte und starke analgetische Wirkung. Ausserdem ist auch eine blutdrucksenkende Wirkung vorhanden.

Der Erfindung liegt also die Aufgabe zugrunde, Verbindungen mit günstigen pharmakologischen Eigenschaften zur Verfügung zu stellen, die beispielsweise als analgetisch wirkende Arzneimittel verwertbar sind.

Die folgenden Erläuterungen stellen erfindungswesentliche Angaben dar:

Die in der Formel I vorkommenden Alkylgruppen, Alkenylgruppen, Alkinylgruppen, Alkoxygruppen, Alkanoylaminogruppen oder Alkanoylgruppen können gerade oder verzweigt sein.

Dasselbe gilt auch für Alkyl- und Alkoxygruppen, falls diese Bestandteil anderer zusammengesetzter Gruppen sind (zum Beispiel in Form einer Monoalkyl- oder Dialkylaminogruppe, Alkanoylaminogruppe, Alkoxycarbonylaminogruppe, Carbalkoxygruppe, Alkylcarbonylgruppe und ähnlichen Gruppen. Desgleichen kann bei der Bedeutung Phenyl-$C_1$–$C_6$-alkylrest(gruppe) der Alkylteil, falls dieser aus 2–4 C-Atomen besteht, ebenfalls gerade oder verzweigt sein. Bei den Halogenatomen handelt es sich um Chlor, Brom oder Fluor, insbesondere Chlor und Fluor. Die Alkyl- und Alkoxygruppen als solche oder als Bestandteil von anderen zusammengesetzten Gruppen bestehen insbesondere aus 1–4 C-Atomen, vorzugsweise 1 oder 2 C-Atomen. Die Alkenylgruppen beziehungsweise Alkinylgruppen bestehen vorzugsweise aus 3 oder 4 C-Atomen. Alkanoylgruppen oder Alkanoylaminogruppen bestehen insbesondere aus 2–4, vorzugsweise 2–3 C-Atomen. Der Alkylteil des Phenyl-$C_1$–$C_4$-alkylrestes(gruppe) besteht insbesondere aus 1–3, vorzugsweise 1 oder 2 C-Atomen. Die $C_3$–$C_7$-Cycloalkylgruppe besteht insbesondere aus 5 bis 6 C-Atomen, die $C_5$–$C_7$-Cycloalkenylgruppe insbesondere aus 5 bis 6 C-Atomen. Die $C_2$–$C_4$-Alkylendioxygruppe besteht insbesondere aus 2 bis 3 C-Atomen. Die Gruppe

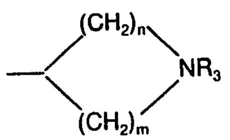

bildet insbesondere einen 5-, 6- oder 7-Ring.

Beispiele hierfür sind: Piperidinring (Piperidyl-(4)-, Piperidyl-(3)- oder Piperidyl-(2)-ring), Homopiperidinring (zum Beispiel Homopiperidyl-(4)-ring), Pyrrolidinring (Pyrrolidyl-(2)- oder Pyrrolidyl-(3)-ring).

Als Chinuclidinring kommt vorzugsweise der Chinuclidyl-(3)-rest, als Tropanylring der Tropanyl-(3)-rest in Frage.

X bedeutet vorzugsweise Schwefel.

Besonders wichtig sind solche Verbindungen der Formel I, worin X Schwefel, einer der Reste $R_1$ beziehungsweise $R_2$ Wasserstoff, der gesättigte stickstoffhaltige Ring ein Piperidylrest ist, der direkt mit dem Schwefelatom verbunden ist (Alk = 0 C-Atome, das heisst Alk entfällt) und $R_3$ Wasserstoff, eine $C_3$–$C_6$-Alkenylgruppe (gerade oder verzweigt) oder eine gerade oder verzweigte $C_1$–$C_6$-Alkylgruppe, die am endständigen C-Atom auch 2 $C_1$–$C_4$-Alkoxygruppen oder eine $C_2$–$C_4$-Alkylendioxygruppe enthalten kann, darstellt. Hierbei enthält der Pyridinring vorzugsweise einen Substituenten entsprechend den angegebenen Bedeutungen für $R_1/R_2$, vorzugsweise ist dieser Substituent ein Halogenatom (beispielsweise Chlor), welches sich insbesondere in 6-Stellung des Pyridinringes befindet. Falls Alk vorhanden ist, besteht diese Gruppe insbesondere aus 1 oder 2 C-Atomen.

Das Verfahren zur Herstellung von Verbindungen der Formel I aus Verbindungen der Formel II und III wird in einem Lösungs- oder Dispergiermittel bei Temperaturen zwischen 20 und 200 °C, vorzugsweise 40 und 150 °C, insbesondere 50 und 120 °C durchgeführt. Als Lösungs- beziehungsweise Dispergiermittel kommen zum Beispiel in Frage: niedere aliphatische Alkohole (1–6 C-Atome); Propanol, Isopropanol, Butanol, niedere aliphatische Ether (Diethylether, Diisopropylether), aromatische Kohlenwasserstoffe (Benzol, Toluol, Xylol), cyclische Ether (Dioxan, Tetrahydrofuran), Ester von niederen aliphatischen Carbonsäuren mit niederen aliphatischen Alkoholen, Amide und N-alkyl-substituierte Amide von aliphatischen $C_1$–$C_4$-Carbonsäuren (Dimethylformamid, Dimethylacetamid), $C_1$–$C_6$-Dialkylsulfone (Dimethylsulfon, Tetramethylensulfon), $C_1$–$C_6$-Dialkylsulfoxide (Dimethylsulfoxid) sowie weitere aprotische Mittel wie N-Methylpyrrolidon, Tetramethylharnstoff, Hexamethylphosphorsäuretriamid, Acetonitril. Die einzelnen Alkylreste der oben angegebenen Lösungsmittel enthalten beispielsweise 1–6, insbesondere 1–4 Kohlenstoffatome.

Das Verfahren wird zweckmässig in Gegenwart von Kondensationsmitteln durchgeführt. Als derartige Kondensationsmittel kommen zum Beispiel in Frage: anorganische Kondensationsmittel wie Alkali- oder Erdalkalihydroxide, Alkalihydride, Alkaliamide, Alkali- oder Erdalkalicarbonate oder organische Basen wie Pyridin, tertiäre Amine, Piperidin, Alkalialkoholate, Alkaliacetate oder auch Triethylphosphat. Bei den Alkalimetallen handelt es sich insbesondere um Natrium oder Kalium. Es kann auch unter Phasen-Transfer-Bedingungen (das heisst unter Zusatz eines oder mehrerer langkettiger Amine wie einem Benzyltributylammonium-halogenid, einem Tetrabutyl-ammonium-halogenid oder Benzyl-triphenyl-phosphoniumchlorid) gearbeitet werden.

Im allgemeinen stellt man aus der Ausgangskomponente, die die Hydroxy- beziehungsweise Mercaptogruppe enthält, zuerst unter Verwendung einer wie oben angegebenen Alkaliverbindung das entsprechende Salz her und setzt dieses dann anschliessend mit der zweiten Reaktionskomponente um. Es kann auch eine Ausgangskomponente der Formel III verwendet werden, die anstelle des Restes $R_3$ eine übliche Amino-Schutzgruppe enthält, welche nach Beendigung der Reaktion leicht solvolytisch oder hydrierend abspaltbar ist. Einige Bedeutungen von $R_3$ stellen bereits solche Schutzgruppen dar (zum Beispiel Benzyl, Alkoxycarbonyl). Bei solchen Gruppen ist eine nachträgliche Abspaltung natürlich nicht zwingend erforderlich.

Falls Y der Formel III eine $C_1$–$C_6$-Alkyl-sulfonyloxygruppe ist, handelt es sich vorzugsweise um eine solche mit 1–4 C-Atomen im Alkylteil (beispielsweise die Methylsulfonyloxygruppe). Falls Y der Formel III eine Arylsulfonyloxygruppe ist, handelt es sich bei dem Arylrest vorzugsweise um einen Phenyl- oder Naphthylrest, wobei diese gegebenenfalls durch $C_1$–$C_4$-Alkylreste (insbesondere Methylreste) substituiert sein können (zum Beispiel p-Toluolsulfonyloxygruppe).

Herstellung von Ausgangsstoffen der Formel II, worin Z = SH ist: Solche Verbindungen können beispielsweise aus Verbindungen der Formel II, worin Z ein Halogenatom (Fluor, Chlor, Brom, Jod)

ist, durch Umsetzen mit Natrium- oder Kaliummercaptid in Alkoholen (Methanol, Ethanol, Propylenglykol) bei Temperaturen zwischen 20 und 150 °C oder auch in wässrigem Medium bei 100–150 °C oder durch Umsetzung mit Thioharnstoff in niederen Alkoholen (Ethanol, Isopropanol) bei Temperaturen zwischen 20 und 100 °C und anschliessender alkalischer Zersetzung (beispielsweise mit wässrigem Natriumcarbonat auf dem Dampfbad) erhalten werden. Eine weitere Möglichkeit ist das Erhitzen von Verbindungen der Formel II

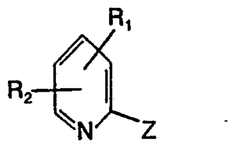

worin Z eine Hydroxygruppe ist mit Phosphorpentasulfid auf Temperaturen zwischen 50 und 200 °C, zum Beispiel 60–160 °C. Diese Umsetzungen können analog den Verfahren erfolgen, die beispielsweise in Erwin Klingenberg, Pyridine and Its Derivates, Part IV (1964), Seiten 348–351 oder der DE-OS 2 230 392, Seite 9 angegeben sind.

Ausgangsstoffe der Formel III

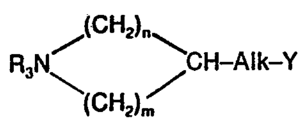

worin Y die Hydroxygruppe und $R_3$ eine andere Bedeutung als Wasserstoff hat, können aus solchen Verbindungen der Formel III, worin $R_3$ Wasserstoff ist, durch Einführung des Restes $R_3$ durch N-Alkylierung, N-Acylierung sowie Anlagerung entsprechend α-β-ungesättigter Verbindungen in an sich bekannter Weise oder gemäss den Bedingungen erhalten werden, die in dieser Anmeldung für die Einführung des Restes $R_3$ in Verbindungen der Formel I mit $R_3$ = H angegeben sind. Solche Verbindungen der Formel III, worin $R_3$ Wasserstoff ist, können beispielsweise aus Verbindungen der Formel III, worin $R_3$ eine Methylgruppe darstellt (die übrigen Reste beziehungsweise Symbole die angegebenen Bedeutungen haben können) durch Umsetzen mit Chlorameisensäureethylester und anschliessender Abspaltung der Carbethoxygruppe erhalten werden (die Reaktionsbedingungen sind die gleichen, die in dieser Anmeldung für die analogen Umsetzungen von Verbindungen der Formel I beschrieben werden.

Aus Verbindungen der Formel III, worin Y die Hydroxygruppe ist, können solche Ausgangsstoffe der Formel III erhalten werden, worin Y ein Halogenatom ist und zwar beispielsweise durch Umsetzung mit Thionylhalogeniden (Chloriden, Bromiden, Jodiden) oder Sulfonsäurechloriden in Halogenkohlenwasserstoffen (Chloroform) oder aromatischen Kohlenwasserstoffen (Benzol) oder in Pyridin bei Temperaturen zwischen 20 und 150 °C (vorzugsweise Siedetemperatur des verwendeten Lösungsmittels).

Ausgangsstoffe der Formel III, worin Y eine Alkylsulfonyloxygruppe oder eine Arylsulfonyloxygruppe ist, können zum Beispiel aus den entsprechenden Hydroxyverbindungen (X = OH) durch Umsetzung mit $C_1$–$C_6$-Alkyl-sulfonsäurechloriden oder den entsprechenden Arylsulfonsäurechloriden in hierfür üblichen inerten Lösungsmitteln (Benzol, Toluol, Xylol, Chloroform, Methylenchlorid, Dioxan) bei Temperaturen zwischen 20–150 °C erhalten werden. Zweckmässig wird hierbei in Gegenwart einer säurebindenden Substanz (zum Beispiel tertiäre Amine wie Triethylamin) gearbeitet. Falls $R_3$ Wasserstoff ist, kann das N-Atom durch eine leicht abspaltbare Schutzgruppe geschützt werden.

Auch den Halogeniden der Formel III (Y = Halogen) können beispielsweise durch Umsetzung mit Alkalisulfiden Ausgangsstoffe der Formel III erhalten werden, worin Y die Mercaptogruppe ist. Diese Umsetzungen können analog C. Ferri, Reaktionen der organischen Synthese 1978, Seiten 205–209 oder analog der DE-OS 2 230 392 zum Beispiel Seite 9 erfolgen.

Ausgangsstoffe der Formel III mit folgender Struktur

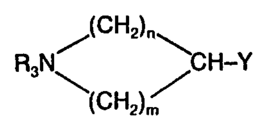 IIIa

(Y = OH, Halogen, SH)

können zum Beispiel wie folgt erhalten werden:

In eine Verbindung der Formel IIIa, worin $R_3$ Wasserstoff ist und die Gruppierung >CHY die Struktur >C=O hat, wird der Rest $R_3$ analog wie auf den folgenden Seiten beschrieben ist durch Alkylierung beziehungsweise Acylierung eingeführt; die so erhaltene Verbindung

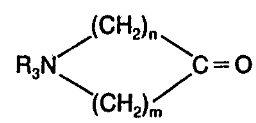 IIIb

kann dann analog H. Barrera und R.E. Lyle, J. Org. Chem. 27 (1962), Seiten 641–643 mit Schwefelwasserstoff umgesetzt und anschliessend mit Natriumborhydrid zur Verbindung IIIa, worin Y = SH ist, reduziert werden.

Man kann aber auch in einer Verbindung IIIb die Ketogruppe in bekannter Weise mit Alkaliboranaten (Na, K, Li) oder anderen komplexen Metallhydriden (zum Beispiel Lithiumaluminiumhydrid) zur Hydroxygruppe reduzieren (siehe Houben-Weyl, Methoden der Organischen Chemie, Band 4/1d, 1981, Seite 271ff.), die Hydroxygruppe mittels üblicher Chlorierungsmittel (zum Beispiel Thionylchlorid, Sulfurylchlorid) gegen ein Chloratom austauschen (siehe Houben-Weyl, Methoden der Organischen Chemie, Band 5/3, 1962, Seiten 862–912), aus dem so erhaltenen Chlorid mit Magnesium die entsprechende Grignard-Verbindung (Formel IIIa Y = MgCl) herstellen (siehe Houben-Weyl, Methoden der Organischen Chemie, Band 13/2a, 1973, Seiten 53–85) und aus einer solchen Grignard-Verbindung mittels Schwefel oder Thionylchlorid (siehe Houben-Weyl, Methoden der Organischen Chemie, Band 9, 1955, Seite 19; E.E. -

Reid, Organic Chemistry of Bivalent Sulfur Bd. I Chem. Publ. Corp., New York, 1958, Seite 37) die Mercapto-Verbindung IIIa, worin Y = SH ist, herstellen.

Falls gewünscht, kann der Rest $R_3$ (Methylrest oder Acylrest) wieder in üblicher Weise abgespalten werden.

Ausgangsstoffe der Formel IIIa, worin Y eine Hydroxygruppe und $R_3$ Wasserstoff ist, können auch beispielsweise aus den entsprechenden Pyridinolen durch Reduktion mit Natrium oder katalytisch aktivierten Wasserstoff (gegebenenfalls unter Druck, zum Beispiel bis 100 bar) bei Temperaturen zwischen 20 und 150 °C erhalten werden (Lösungsmittel: $C_1$–$C_6$-Alkohole). In diese Verbindungen kann dann wie nachstehend beschrieben der Rest $R_3$ eingeführt werden.

Die Überführung der Reste $R_1$, $R_2$ und $R_3$ von Verbindungen der Formel I in andere Bedeutungen kann beispielsweise durch folgende Reaktionen erfolgen:

1. Durch Alkylierung beziehungsweise Acylierung. Insbesondere handelt es sich hier um die Einführung des Restes $R_3$ in Verbindungen der Formel I, worin $R_3$ = Wasserstoff ist, aber auch um die Acylierung oder Alkylierung von Aminogruppen (zum Beispiel wenn $R_1$ und/oder $R_2$ Aminogruppen darstellen). Die Alkylierung erfolgt beispielsweise durch Umsetzung mit Verbindungen der Formel R'Hal, $ArSO_2OR'$ und $SO_2(OR'_3)_2$, wobei Hal ein Halogenatom (insbesondere Chlor, Brom oder Jod) und Ar ein aromatischer Rest (zum Beispiel ein gegebenenfalls durch einen oder mehrere niedere Alkylreste substituierter Phenyl- oder Naphthylrest und R' die für $R_3$ genannten Bedeutungen (ausgenommen Wasserstoff) haben kann. Beispiele sind
p-Toluolsulfonsäure-$C_1$–$C_6$-alkylester,
$C_1$–$C_6$-Dialkylsulfate, $C_1$–$C_6$-Alkylhalogenide,
$C_3$–$C_6$-Alkenylhalogenide, $C_3$–$C_6$-Alkinylhalogenide,
$C_3$–$C_7$-Cycloalkylhalogenide,
$C_5$–$C_7$-Cycloalkenylhalogenide und ähnliche.
Bei den zuvor genannten Verbindungen kann die Alkylgruppe jeweils entsprechend der Bedeutung von $R_3$ substituiert sein. Falls $R_3$ eine $C_1$–$C_6$-Alkylgruppe ist, die mindestens eine Hydroxygruppe (in 2-Stellung) enthält, kann die Alkylierung auch durch eine entsprechende $C_1$–$C_6$-Alkylenoxid-Verbindung erfolgen, die natürlich noch weitere Substituenten entsprechend der Bedeutung von $R_3$ enthalten kann. Die Alkylierungs- und Acylierungsreaktion wird gegebenenfalls unter Zusatz von üblichen säurebindenden Mitteln, wie Alkalihydroxiden, Alkalicarbonaten, Alkalihydrogencarbonaten, Erdalkalicarbonaten, Alkaliacetaten, tertiären Aminen (zum Beispiel Trialkylamin wie Triethylamin), Pyridin oder auch Alkalihydriden bei Temperaturen zwischen 0 und 200 °C, vorzugsweise 40 und 140 °C in inerten Lösungsmitteln oder Suspensionsmitteln durchgeführt. Als Lösungs- oder Dispergiermittel kommen beispielsweise in Betracht: aromatische Kohlenwasserstoffe wie zum Beispiel Benzol, Toluol, Xylol; aliphatische Ketone wie zum Beispiel Aceton, Methylethylketon; halogenierte Kohlenwasserstoffe wie zum Beispiel Chloroform, Tetrachlorkohlenstoff, Chlorbenzol, Methylenchlorid; aliphatische Ether wie zum Beispiel Butylether; cyclische Ether wie zum Beispiel Tetrahydrofuran, Dioxan; Sulfoxyde wie zum Beispiel Dimethylsulfoxyd; tertiäre Säureamide wie zum Beispiel Dimethylformamid, N-Methylpyrrolidon, Hexamethylphosphorsäuretriamid; aliphatische Alkohole wie Methanol, Ethanol, Isopropanol, Amylalkohol, tert.-Butanol, cycloaliphatische Kohlenwasserstoffe wie Cyclohexan und ähnliche. Auch wässrige Mischungen der genannten Lösungsmittel können verwendet werden. Häufig arbeitet man bei der Rückflusstemperatur der verwendeten Lösungs- beziehungsweise Dispergiermittel. Häufig werden die Alkylierungsreaktionskomponenten im Überschuss eingesetzt. Die Alkylierung kann auch in Gegenwart von Tetraalkylammoniumsalzen (insbesondere der Halogenide) in Kombination mit Alkalihydroxiden bei Temperaturen zwischen 0–100 °C, vorzugsweise 20–80 °C in einem aprotischen Lösungsmittel oder auch in Chloroform oder Methylenchlorid vorgenommen werden. Als aprotische Lösungsmittel kommen insbesondere in Betracht: tertiäre Amide (Dimethylformamid, N-Methyl-Pyrrolidon, Hexamethylphosphorsäuretriamid), Dimethylsulfoxid, Acetonitril, Dimethoxyethan, Aceton, Tetrahydrofuran.

Bei der Acylierung werden zum Beispiel in Verbindungen der Formel I, worin $R_3$ Wasserstoff ist, eine gegebenenfalls durch einen $C_3$–$C_6$-Cycloalkylrest substituierte $C_2$–$C_6$-Alkanoylgruppe oder eine Carb-$C_1$–$C_6$-alkoxygruppe eingeführt. Man verfährt hierbei in an sich bekannter Weise vorzugsweise unter Verwendung von Carb-$C_1$–$C_6$-alkoxyhalogeniden (oder der entsprechenden Anhydride) oder unter Verwendung von $C_2$–$C_6$-Alkanoylhalogeniden (beziehungsweise entsprechender Anhydride), wobei die Alkanoylgruppe auch durch einen $C_3$–$C_6$-Cycloalkylrest substituiert sein kann. Die Reaktionstemperaturen liegen vorzugsweise zwischen 30 und 120 °C.

Gegebenenfalls kann man bei der Alkylierung und der Acylierung auch so vorgehen, dass man zuerst von der zu alkylierenden beziehungsweise acylierenden Verbindung eine Alkaliverbindung (Natrium-, Kalium- oder auch Lithiumsalz zum Beispiel) herstellt, indem man sie in einem inerten Lösungsmittel wie Dioxan, Dimethylformamid, Benzol oder Toluol mit einem Alkalimetall, Alkalihydrid oder Alkaliamiden (insbesondere Natrium oder Natriumverbindungen) oder Butyllithium bei Temperaturen zwischen 0 und 150 °C umsetzt und dann das alkylierende Agens zufügt.

Anstelle der angeführten Alkylierungs- und Acylierungsmittel können auch andere in der Chemie gebräuchliche chemisch-äquivalente Mittel verwendet werden (siehe zum Beispiel auch L.F. und Mary Fieser «Reagents for Organic Synthesis», John Wiley and Sons, Inc., New York, 1967, Vol. 1, Seiten 1303–4 und Vol. 2, Seite 471).

2. An Verbindungen der Formel I, worin $R_3$ Wasserstoff ist, können zum Beispiel $C_3$–$C_6$-Alkenyle, die in Nachbarstellung zur Doppelbindung eine

$C_1$–$C_6$-Alkylcarbonylgruppe, eine Carb-$C_1$–$C_6$-alkoxygruppe oder eine Benzoylgruppe enthalten, angelagert werden. Diese Reaktion kann in Lösungsmitteln bei Temperaturen zwischen 20–150 °C durchgeführt werden. Als Lösungsmittel eignen sich hierfür zum Beispiel: $C_1$–$C_6$-Alkanole, aliphatische gesättigte Ether, aromatische Kohlenwasserstoffe (Benzol, Toluol, Xylole) chlorierte aliphatische Kohlenwasserstoffe (Methylenchlorid, Chloroform, Dichlorethan).

3. In solche Verbindungen der Formel I, worin $R_3$ eine Methylgruppe ist, kann durch Reaktion mit Halogenameisensäure-$C_1$–$C_6$-alkylestern oder mit Phosgen, gefolgt von einer anschliessenden Umsetzung mit einem $C_1$–$C_6$-Alkanol, die Carb-$C_1$–$C_6$-Alkoxygruppe eingeführt werden. Vorzugsweise werden Chlorameisensäure-$C_1$–$C_6$alkylester verwendet (zum Beispiel Chlorameisensäureethylester).

Diese Reaktionen werden mit oder ohne Lösungs- beziehungsweise Suspensionsmittel bei Temperaturen zwischen 20 bis 180 °C, vorzugsweise 40 bis 120 °C durchgeführt. Als Lösungsmittel für diese Reaktionen kommen beispielsweise in Frage: aromatische Kohlenwasserstoffe (Toluol, Xylol), chlorierte Kohlenwasserstoffe wie Methylenchlorid oder Chloroform.

4. Falls in Verbindungen der Formel I der Rest $R_3$ eine Carb-$C_1$–$C_6$-alkoxygruppe, eine $C_2$–$C_6$-Alkanoylgruppe (gegebenenfalls durch einen Cycloalkylrest substituiert) ist, können diese Gruppen solvolytisch abgespalten werden unter Bildung von Verbindungen der Formel I, worin $R_3$ Wasserstoff ist. Diese Abspaltung erfolgt in bekannter Weise beispielsweise durch Verseifung mit Säuren (Mineralsäuren wie Salzsäure, Schwefelsäure, insbesondere konzentrierten Halogenwasserstoffsäuren wie HBr/Eisessig) oder mittels basischer Substanzen (Pottasche, Soda, wässrige Alkalilösungen, alkoholische Alkalilösungen, wässriges $NH_3$) bei Temperaturen zwischen 10 und 150 °C, insbesondere 20–100 °C. Falls $R_3$ die Gruppe S ist und diese Gruppe S eine solvolytisch abspaltbare Schutzgruppe ist (zum Beispiel: Trifluoracetylrest, Tritylrest, p-Toluolsulfonylrest, Formylrest, tert.-Butyloxicarbonylrest und ähnliche), erfolgt die Abspaltung dieser Gruppe S in gleicher Weise.

Falls $R_3$ eine Benzylgruppe, eine α-Phenylethylgruppe oder als Gruppe S eine andere übliche hydrierend abspaltbare Schutzgruppe darstellt, erfolgt die Abspaltung zweckmässig durch katalytische Hydrierung in Gegenwart üblicher Hydrierungskatalysatoren, insbesondere Palladium-Katalysatoren, Platinoxyd oder auch Raney-Nickel, in einem Lösungs- ode Suspensionsmittel, gegebenenfalls unter erhöhtem Druck bei Temperaturen zwischen 20–100 °C, insbesondere 40–80 °C. Als Lösungs- beziehungsweise Suspensionsmittel kommen beispielsweise in Betracht: Wasser, niedere aliphatische Alkohole, cyclische Ether wie Dioxan oder Tetrahydrofuran, aliphatische Ether, Dimethylformamid und so weiter sowie Mischungen dieser Mittel. Als Schutzgruppen, die durch Hydrogenolyse abspaltbar sind, kommen beispielsweise in Frage: α-Arylalkylreste, im Benzolkern substituierte Benzylreste (p-Brom- oder p-Nitrobenzylrest), Aralkoxycarbonylreste wie Carbobenzoxyrest, Carbobenzthiorest.

Als Schutzgruppen S kommen insbesondere die bei der Peptid-Synthese üblichen Schutzgruppen in Frage. Unter anderem wird hierzu auch auf das Buch von Jesse P. Greenstein und Milton Winitz «Chemistry of Amino Acids», N.Y. 1961, John Wiley und Sons, Inc. Volume 2, beispielsweise Seite 883ff. verwiesen.

5. Falls einer oder auch beide der Reste $R_1$, $R_2$ eine Nitrogruppe bedeuten, kann diese zur entsprechenden Aminogruppe reduziert werden. Für diese Reduktion kommt insbesondere die katalytische Hydrierung in Betracht. Als Katalysatoren kommen zum Beispiel in Frage: Raney-Nickel, Edelmetalle, wie Palladium und Platin sowie Verbindungen davon mit und ohne Träger wie beispielsweise Bariumsulfat, Calciumsulfat und so weiter. Es empfiehlt sich, die Hydrierung der Nitrogruppe bei Temperaturen zwischen 20 und 80 °C und einem Druck von ungefähr 5–50 atü in einem Lösungsmittel, beispielsweise Alkoholen, Dioxan, Tetrahydrofuran und so weiter vorzunehmen. Für die anschliessende Isolierung der reduzierten Verbindungen kann es in manchen Fällen von Vorteil sein, wenn zu Beginn dem zu hydrierenden Gemisch Trockenmittel, wie wasserfreies Natrium- oder Magnesiumsulfat zugesetzt werden. Die Reduktion kann aber auch mit nascierendem Wasserstoff, beispielsweise Zink/Salzsäure, Zinn/Salzsäure, Eisen/Salzsäure oder mit Salzen des Schwefelwasserstoffs in Alkohol/Wasser bei etwa 70 bis etwa 120 °C oder mit aktiviertem Aluminium in wasserhaltigem Ether bei 20 bis 40 °C oder mit Zinn(II)-chlorid/Salzsäure durchgeführt werden.

6. Aktivierte Halogenatome im Pyridinring können zum Beispiel gegen andere Reste, beispielsweise eine Mono- oder Di-$C_1$–$C_6$-alkylaminogruppe, oder eine Aminogruppe, die gegebenenfalls durch Phenyl oder Halogenphenyl oder Phenyl-$C_1$–$C_4$-alkyl substituiert ist, ausgetauscht werden. Diese Umsetzung kann beispielsweise in einem inerten Lösungs- oder Suspensionsmittel wie Tetrahydrofuran, Dioxan, niederen Alkanolen (Ethanol, n-Propanol), Dimethylsulfoxid oder Dimethylformamid, oder auch in Gegenwart eines Überschusses des basischen Reaktionspartners bei Temperaturen zwischen 50 bis 200 °C, vorzugsweise 80 bis 130 °C, durchgeführt werden. Dabei können Säureakzeptoren wie Pottasche, Natriumkarbonat, Calziumcarbonat oder nicht quaternisierende tertiäre Amine, wie Diisopropylmethylamin, zugesetzt werden. Halogenatome in 3-, 4- oder 5-Stellung des Pyridinringes eignen sich hierfür, wenn sie zum Beispiel durch eine Nitrogruppe aktiviert sind.

Die Überführung von Verbindungen der Formel I in die entsprechenden Pyridin-N-oxide kann beispielsweise in inerten Lösungsmitteln wie Chloroform oder anderen Chlorkohlenwasserstoffen, Benzol, Toluol, Aceton, verdünnter Essigsäure oder Essigsäureäthylester mit Wasserstoffperoxyd, einer üblichen aliphatischen oder aromatischen Persäure (Peressigsäure, Benzopersäure, m-Chlorbenzopersäure) oder anderen Monosubstitutionsprodukten des Wasserstoffperoxyds wie Alkaliperoxiden oder Alkylperoxiden (zum Beispiel tert.-Butylperoxid) bei Temperaturen zwischen 0 und 150 °C, vorzugsweise 0 bis 100 °C, durchgeführt werden. Falls X = S ist, entstehen hierbei zuerst die entsprechenden Sulfoxide beziehungsweise Sulfone. Diese lassen sich dann jedoch weiter zu den Aminoxiden oxydieren.

Die Überführung von Verbindungen der Formel I, worin X ein Schwefelatom bedeutet in solche Verbindungen, worin X die Gruppe SO oder $SO_2$ ist, erfolgt ebenfalls durch Oxydation in an sich bekannter Weise. Als Oxydationsmittel können mit gutem Erfolg zum Beispiel Wasserstoffperoxyd, Distickstofftetroxid, Kaliumpermanganat, Persäuren (zum Beispiel Benzopersäure, Phthalmonopersäure, Peressigsäure) Salpetersäure, Chromsäure oder andere bekannte Oxydationsmittel verwendet werden. Hierbei arbeitet man zweckmässig in Gegenwart von Wasser oder von Lösungsmitteln, zum Beispiel von Alkoholen, Essigsäure (Eisessig), Essigsäureäthylester, Benzol, Aceton oder Chloroform. Besonders die niederen Alkohole, zum Beispiel Methanol oder auch Essigsäure sind gut geeignet. Bei der Oxydation mit 30%igem Wasserstoffperoxid, Persäuren, Salpetersäure, nitrosen Gasen (Stickstoffdioxid) unter Kühlung, beispielsweise bei Temperaturen zwischen −20° und +20 °C erhält man im allgemeinen als Hauptprodukt das entsprechende Sulfoxid neben geringeren Mengen des Sulfons. Weiterhin können entsprechende Sulfoxide aus Verbindungen der Formel I, worin X = S ist, durch Oxydation mit Chromsäure (zum Beispiel in essigsaurer Lösung bei Temperaturen zwischen 50–100 °C), durch Oxydation mit beispielsweise Jodosobenzol oder durch Behandlung mit Brom (zum Beispiel in einem Halogenkohlenwasserstoff wie Chloroform oder Tetrachlorkohlenstoff unter Kühlung) und anschliessender Hydrolyse der Dibromderivate mittels Wasser oder verdünnter Alkalilauge hergestellt werden. Hinsichtlich der Reaktionsbedingungen sowie anderer Oxydationsmittel wird zum Beispiel auf Houben, Weyl, Methoden der Organischen Chemie, Band IX (1955), Seiten 211–218 verwiesen. Auch eine Oxydation von Sulfiden der Formel I (X = S) mit Dimethylsulfoxid bei höherer Temperatur (150–180 °C) gemäss J. Org. Chem. 23 (1958), Seiten 2028–2029 ist möglich.

Die jeweils erhaltenen Sulfone und Sulfoxide können mittels üblicher Trennverfahren, beispielsweise durch Säulenchromatographie an Kieselgel getrennt werden.

Mit stärkeren Oxydationsmitteln wie zum Beispiel Kaliumpermanganat in essig- oder wässrigschwefelsaurer Lösung bei Temperaturen zwischen 50 und 100 °C erhält man das entsprechende Sulfon in grösserer Ausbeute beziehungsweise als Hauptprodukt. Die Oxydation von Verbindungen der Formel I worin X = S oder SO ist, kann beispielsweise auch mittels Wasserstoffperoxid oder Persäuren bei höherer Temperatur, wie zum Beispiel 80–120 °C (in essigsaurer Lösung oder in Eisessig und Essigsäureanhydrid, in Anwesenheit von Phosphorsäure oder einem sonstigen hierfür üblichen inerten Mittel), mittels Chromsäure, mittels anodischer Oxydation oder gegebenenfalls auch mittels Natriumhypochloritlösungen erfolgen (siehe Houben-Weyl, Methoden der Organischen Chemie, Band IX (1955), Seiten 227–231). Eine weitere Möglichkeit ist die Oxydation mit organischen Hydroperoxiden (zum Beispiel Alkylhydroperoxiden wie tert.-Butylhydroperoxid) in Gegenwart von Vanadin-, Molybdän- oder Titanverbindungen (zum Beispiel Oxide der genannten Metalle wie Molybdändioxid, Vanadinpentoxid) in organischen Lösungsmitteln wie aromatischen Kohlenwasserstoffen (Benzol), Alkanolen (Ethanol) oder Estern aliphatischer Carbonsäuren mit Alkanolen (Ethylacetat) bei Temperaturen zwischen 40–120 °C, vorzugsweise 50–80 °C gemäss Angewandte Chemie 78 (1966), Seite 937.

Diejenigen Verbindungen der Formel I, die asymmetrische Kohlenstoffatome enthalten und in der Regel als Razemate anfallen, können in an sich bekannter Weise beispielsweise mit Hilfe einer optisch aktiven Säure in die optisch aktiven Isomeren gespalten werden. Es ist aber auch möglich, von vornherein eine optisch aktive Ausgangssubstanz einzusetzen, wobei dann als Endprodukt eine entsprechende optisch aktive beziehungsweise diastereomere Form erhalten wird.

Die vorliegende Erfindung umfasst also auch die D- und L-Form wie auch die DL-Mischung für den Fall, dass in der Verbindung der Formel I ein asymmetrisches C-Atom vorkommt und für den Fall von zwei und mehr asymmetrischen C-Atomen ebenso die entsprechenden diastereomeren Formen.

Je nach den Verfahrensbedingungen und Ausgangsstoffen erhält man die Endstoffe der Formel I in freier Form oder in Form ihrer Salze. Die Salze der Endstoffe können in an sich bekannter Weise, beispielsweise mit Alkali oder Ionenaustauschern wieder in die Basen übergeführt werden. Von den letzteren lassen sich durch Umsetzung mit organischen oder anorganischen Säuren, insbesondere solchen, die zur Bildung von therapeutisch verwendbaren Salzen geeignet sind, Salze gewinnen. Als solche Säuren seien beispielsweise genannt: Halogenwasserstoffsäuren, Schwefelsäure, Phosphorsäuren, Salpetersäure, Perchlorsäure, organische Mono-, Di- oder Tricarbonsäuren der aliphatischen, alicyclischen, aromatischen oder heterocyclischen Reihe sowie Sulfonsäuren. Beispiele hierfür sind:
Ameisen-, Essig-, Propion-, Bernstein-,
Glykol-, Milch-, Äpfel-, Wein-, Zitronen-,
Ascorbin-, Malein-, Fumar-, Hydroxymalein-
oder Brenztraubensäure; Phenylessig-, Benzoe-,
p-Aminosalicylsäure, Embonsäure,
Methansulfon-, Ethansulfon-,
Hydroxyethansulfon-, Ethylensulfonsäure;
Halogenbenzolsulfon-, Toluolsulfon-,
Naphthalinsulfonsäure oder Sulfanilsäure
oder auch 8-Chlor-theophyllin.

Die erfindungsgemässen Verbindungen sind zur Herstellung pharmazeutischer Zusammensetzungen geeignet. Die pharmazeutischen Zusammensetzungen beziehungsweise Medikamente können eine oder mehrere der erfindungsgemässen Verbindungen enthalten. Zur Herstellung der pharmazeutischen Zubereitungen können die üblichen pharmazeutischen Träger- und Hilfsstoffe verwendet werden. Die Arzneimittel können zum Beispiel enteral, parenteral (zum Beispiel intravenös, intramuskulär, subkutan) oder oral angewendet werden. Beispielsweise kann die Verabreichung in Form von Tabletten, Kapseln, Pillen, Dragees, Zäpfchen oder Pflastern erfolgen. Als Liquida kommen zum Beispiel in Frage: Ölige oder wässrige Lösungen oder Suspensionen (zum Beispiel in Sesam- oder Olivenöl), Emulsionen, injizierbare wässrige oder ölige Lösungen oder Suspensionen. Weiterhin können beispielsweise Trockenampullen, welche als Wirkstoff die erfindungsgemässe Verbindung I enthalten, hergestellt werden, wobei vor Gebrauch der Inhalt solcher Trockenampullen zum Beispiel in Wasser, physiologischer Kochsalzlösung oder Gemischen aus physiologischer Kochsalzlösung und beispielsweise Dimethylsulfoxid aufgelöst wird.

Pharmakologische beziehungsweise pharmazeutische Angaben

Die erfindungsgemässen Verbindungen sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemässen Verbindungen, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Encyclopädie der technischen Chemie, Band 4 (1953), Seite 1 bis 39; Journal of Pharmaceutical Sciences, Band 52 (1963), Seite 918 u.ff., H.v. Czetsch-Lindenwald, Hilfsstoffe für Pharmazie und angrenzende Gebiete; Pharm. Ind., Heft 2, 1961, Seite 72 u.ff.; Dr. H.P. Fiedler, Lexikon der Hilfsstoffe für Pharmazie, Kosmetik und angrenzende Gebiete Cantor KG. Aulendorf in Württemberg 1971.

Die pharmazeutische und galenische Handhabung der erfindungsgemässen Verbindungen erfolgt nach den üblichen Standardmethoden. Beispielsweise werden Wirkstoff (e) und Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 80 °C, vorzugsweise 20 bis 50 °C, insbesondere bei Raumtemperatur gearbeitet wird. Im übrigen wird auf das folgende Standardwerk verwiesen: Sucker, Fuchs, Speiser, Pharmazeutische Technologie, Thieme-Verlag Stuttgart, 1978.

Die erfindungsgemässen Verbindungen zeigen zum Beispiel im Elektroschmerz-Test*, Hot-Plate-Test**, Tail-Flick-Test*** und Haffner-Test**** eine gute analgetische Wirkung.

Beispielsweise liegt bei obengenannten Versuchsmethoden die ED 50 bei einer Dosis von 2,8 mg/Körpergewicht Maus per os.

Diese analgetische Wirkung ist mit der Wirkung des bekannten Arzneimittelwirkstoffs Buprenorphin vergleichbar.

Die niedrigste, bereits analgetisch wirksame Dosis in dem oben angegebenen Tierversuch ist beispielsweise

1–1,5 mg/kg oral

0,1–0,15 mg/kg intravenös

Als allgemeiner Dosisbereich für die Wirkung (Tierversuch wie oben) kommt beispielsweise in Frage:

1–8 mg/kg oral

0,1–0,8 mg/kg intravenös

Die pharmazeutischen Zubereitungen enthalten im allgemeinen zwischen 0,1 bis 10 vorzugsweise 0,5 bis 3 mg der erfindungsgemässen aktiven Komponente(n).

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Tabletten, die zwischen 0,5 und 2 mg oder Lösungen, die zwischen 1 bis 10% an aktiver Substanz enthalten.

Die Einzeldosis der erfindungsgemässen aktiven Komponenten kann beispielsweise liegen

a) bei oralen Arzneiformen zwischen 0,1 bis 20 vorzugsweise 0,5 bis 3 mg
b) bei parenteralen Arzneiformen (zum Beispiel intravenös, intramuskulär) zwischen 0,01 bis 1 vorzugsweise 0,05 bis 0,5 mg
c) bei Arzneiformen zur rektalen oder vaginalen Applikation zwischen 0,05 bis 20 mg vorzugsweise 0,05 bis 5 mg.

– (Die Dosen sind jeweils bezogen auf die freie Base) –

Beispielsweise können 3 mal täglich 1 bis 3 Tabletten mit einem Gehalt von 1 bis 3 mg wirksamer Substanz oder zum Beispiel bei intravenöser Injektion 1 bis 3 mal täglich eine Ampulle von 1 bis 10 ml Inhalt mit 0,1 bis 0,5 mg Substanz empfohlen

---

* in Anlehnung an B. Blake et al, Med. exp. 9, Seiten 146–150 (1963)

** in Anlehnung an Janssen und Jageneau, J. Pharm-Pharmacol. 9, Seite 381 (1957)

*** in Anlehnung an D'Amoùr und Smith, J. of Pharmacol. and exp. Therap., 72, Seite 74 (1941)

**** in Anlehnung an Haffner, Deutsche Medizinische Wochenschrift 55, Seite 731 (1929) und Bianchi und Franceschini, Brit. J. Pharmacol. 9, Seite 280 (1954).

werden. Bei oraler Verabreichung ist die minimale tägliche Dosis beispielsweise 1 mg; die maximale tägliche Dosis bei oraler Verabreichung soll nicht über 3 mg liegen.

Für die Behandlung von Hunden und Katzen liegt die orale Einzeldosis im allgemeinen zwischen ungefähr 0,05 und 1 mg/kg Körpergewicht; die parenterale Dosis ungefähr zwischen 0,01 und 0,2 mg/kg Körpergewicht.

Für die Behandlung von Pferden und Vieh liegt die orale Einzeldosis im allgemeinen zwischen ungefähr 0,03 und 0,5 mg/kg; die parenterale Einzeldosis ungefähr zwischen 0,01 und 0,1 mg/kg Körpergewicht.

Die akute Toxizität der erfindungsgemässen Verbindungen an der Maus (ausgedrückt durch die LD 50 mg/kg; Methode nach Miller und Tainter: Proc. Soc. Exper.Biol. a. Med. 57 (1944) 261) liegt beispielsweise bei oraler Applikation zwischen 70 und 110 mg/kg (beziehungsweise oberhalb 80 mg/kg.

Die Arzneimittel können in der Humanmedizin, der Veterinärmedizin sowie in der Landwirtschaft allein oder im Gemisch mit anderen pharmakologisch aktiven Stoffen verwendet werden.

Die efindungsgemässen Verbindungen stellen wirksame Analgetika dar.

Beispiele

Allgemeine Verfahrensvorschrift für die Beispiele 1–21 der Tabelle 1 unter Verwendung einer Ausgangsverbindung II, worin Z ein Halogenatom (Chlor) ist:

0,05 Mol 80%iges Natriumhydrid werden in ca. 30 ml des angegebenen wasserfreien Lösungsmittels (siehe Tabelle 1) suspendiert. Unter Rühren werden bei Raumtemperatur 0,04 Mol des entsprechenden Alkohols der Formel III (Y = OH) oder 0,05 Mol des entsprechenden Mercaptans der Formel III, wobei Y jetzt SH ist (eventuell gelöst in dem gleichen Lösungsmittel), zugetropft. Die Reaktion setzt unter Wasserstoffentwicklung ein. Das Gemisch wird auf 50 °C erwärmt. Falls ein Mercaptan eingesetzt wird, erwärmt man auf 60 °C und löst 0,05 Mol NaH in 50 ml des jeweiligen Lösungsmittels. Nach Beendigung der Reaktion werden 0,05 Mol des entsprechenden Chlorpyridins (eventuell im gleichen absoluten Lösungsmittel, vorzugsweise bei Raumtemperatur, zugetropft und das Reaktionsgemisch einige Stunden (3–6 Stunden) unter Rückfluss erhitzt (im Falle der Verwendung eines Mercaptans auf 80–100 °C). Danach wird nach dem Abkühlen mit Wasser hydrolysiert und die erhaltene wässrige Lösung mit Diethylether oder Methylenchlorid mehrfach extrahiert. Nach Trocknung über Magnesiumsulfat und Filtration wird das Lösungsmittel im Vakuum abdestilliert. Die Aufarbeitung kann in 3 verschiedenen Weisen vorgenommen werden:

A) Reinigung des Rückstandes durch präparative Säulenchromatographie an Kieselgel und eventuelle anschliessende Salzbildung, zum Beispiel mit isopropanolischer HCl;

B) Reinigung durch Destillation im Vakuum und eventuelle anschliessende Salzbildung wie unter A);

C) Ist der erhaltene Rückstand nicht stark verunreinigt, kann die Salzbildung ohne vorhergehende Reinigung vorgenommen werden. In der Regel wird der Rückstand in Isopropanol gelöst und mit isopropanolischer Salzsäure versetzt.

Das auskristallisierte Salz wird abfiltriert und in einem Lösungsmittel umkristallisiert.

Die so hergestellten Verbindungen der untenstehenden Formel sind in der Tabelle 1 aufgeführt.

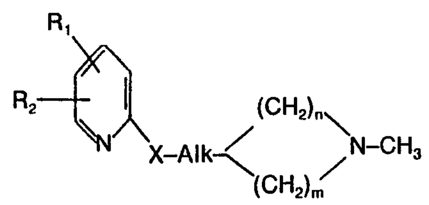

Tabelle 1

| Beispiel Nr. | $R_1$ | $R_2$ | X | Alk | n | m | Lösungsmittel | Reinigungsvariante | F. als Hydrochlorid |
|---|---|---|---|---|---|---|---|---|---|
| 1 | 6-Cl | H | O | – | 2 | 2 | DMSO | C | 186–187°C |
| 2 | 6-Cl | H | O | – | 3 | 1 | DMSO | C | 180–183°C |
| 3 | 6-Cl | H | O | $CH_2CH_2$ | 0 | 3 | DMSO | C | 134–136°C |
| 4 | 6-Cl | H | O | – | 2 | 3 | DMSO | C | 110–113°C |
| 5 | 6-Cl | H | O | $CH_2$ | 0 | 4 | DMAC | C | 147–148°C |
| 6 | 6-Cl | H | O | $CH_2$ | 1 | 3 | DMAC | C | 168–169°C |
| 7 | 6-Cl | 3–$NO_2$ | O | – | 2 | 2 | DMF | C | 214–216°C |

DMSO = Dimethylsulfoxid; DMF = Dimethylformamid; DMAC = Dimethylacetamid
Ein Strich (–) in Spalte Alk bedeutet, dass hier die Gruppe Alk entfällt

Tabelle 1 (Fortsetzung)

| Beispiel Nr. | $R_1$ | $R_2$ | X | Alk | n | m | Lösungsmittel | Reinigungsvariante | F. als Hydrochlorid |
|---|---|---|---|---|---|---|---|---|---|
| 8 | 6-Cl | 3-$NH_2$ | O | – | 2 | 2 | Dioxan | C | 245–250°C |
| 9 | 3-$NO_2$ | H | O | – | 2 | 2 | Dioxan | C | 240–241°C |
| 10 | 6-Cl | 3-$NHCOCH_3$ | O | – | 2 | 2 | Dioxan | C | 226–230°C |
| 11 | 6-Cl | H | O | – | Tropanyl-(3)-Rest | | DMSO | C | 250–251°C |
| 12 | 6-Cl | H | O | – | Chinuclidyl-(3)-Rest | | DMSO | C | 241–243°C |
| 13 | H | H | S | – | 2 | 2 | DMSO | C | 132–134°C Maleat |

DMSO = Dimethylsulfoxid; DMF = Dimethylformamid; DMAC = Dimethylacetamid
Ein Strich (–) in Spalte Alk bedeutet, dass hier die Gruppe Alk entfällt
Die Maleate der Beispiele 14 und 15 wurden mittels isopropanolischer Maleinsäure-Lösung hergestellt

Talbelle 1 (Fortsetzung)

| Beispiel Nr. | $R_1$ | $R_2$ | X | Alk | n | m | Lösungsmittel | Reinigungsvariante | F. als Hydrochlorid |
|---|---|---|---|---|---|---|---|---|---|
| 14 | 6-Cl | H | S | – | 2 | 2 | DMSO | C | 110–112°C Maleat |
| 15 | 6-Cl | H | S | – | 2 | 2 | DMSO | C | 184–187°C |
| 16 | 6-$CH_3$ | H | S | – | 2 | 2 | DMSO | B | 216–218°C Dihydrochlorid |
| 17 | 6-$OCH_3$ | H | S | – | 2 | 2 | DMSO | B | 165–167°C |
| 18 | 6-Br | H | S | – | 2 | 2 | DMSO | C | 198–199°C |
| 19 | 5-Cl | H | S | – | 2 | 2 | Toluol | C | 134–156°C Maleat* |
| 20 | 3-Cl | H | S | – | 2 | 2 | DMSO | C | 125–126°C Maleat* |

DMSO = Dimethylsulfoxid; DMF = Dimethylformamid; DMAC = Dimethylacetamid
Ein Strich (–) in Spalte Alk bedeutet, dass hier die Gruppe Alk entfällt
*Diese Maleate wurden mit Maleinsäure in Aceton hergestellt und aus Ethanol umkristallisiert

Beispiel 21
6-Chlor-2-[(N-2-Phenylethyl)-piperidyl-(4)-oxy]-pyridin

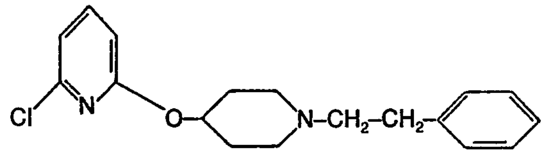

In die Lösung von 8,2 g (0,04 Mol) N-(2-phenylethyl)-4-hydroxypiperidin in 60 ml absolutem Dimethylacetamid werden unter Rühren bei Raumtemperatur portionsweise 1,3 g 75%iges Natriumhydrid eingetragen. Nach beendeter Zugabe werden 5,9 g (0,04 Mol) 2,6-Dichlorpyridin zugefügt. Das Reaktionsgemisch wird 8 Stunden auf 120–130 °C erhitzt. Danach wird auf Raumtemperatur abgekühlt und in ca. 300 ml Wasser eingegossen. Das anfallende kristalline Produkt wird abgetrennt. Nach etwa einstündigem Verrühren in 2 n wässriger Salzsäure wird abgesaugt, mit Wasser gewaschen, getrocknet und aus Ethanol umkristallisiert.
F. des Hydrochlorids: 253–254 °C.

Beispiel 22
6-Chlor-2-[piperidyl-(4)-thio]-pyridin

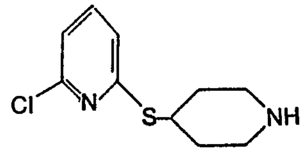

Die Reaktion wird in Argon-Atmosphäre durchgeführt. 0,27 g 80%iges Natriumhydrid (0,009 Mol) werden in 10 ml Dimethylacetamid suspendiert; es wird mit Eis gekühlt und nun 0,615 g (0,004 Mol) festes 4-Mercapto-piperidin-Hydrochlorid zugegeben und 10 Minuten gerührt. Zu dieser Mischung wird dann eine Lösung von 0,588 g (0,004 Mol) 2.6-Dichlor-pyrridin in 5 ml Dimethylacetamid zugetropft und die Reaktionsmischung 2,5 Stunden bei Raumtemperatur gerührt.

Aufarbeitung der Reaktionsmischung: man tropft unter Kühlung 25 ml Wasser zu, gibt anschliessend 20 ml Methylenchlorid zu, trennt die organische Phase ab, schüttelt die wässrige Phase 2 mal mit je 15 ml Methylenchlorid aus, wäscht die vereinigte organische Phase 2 mal mit je 10 ml

Wasser, trocknet mit Natriumsulfat, engt die Lösung am Rotationsverdampfer ein, versetzt den Rückstand mit 10 ml absolutem Ethanol und engt wieder ein. Man erhält ca. 1,5 ml einer gelben Flüssigkeit, die durch Säulenchromatographie über 60 g Kieselgel (Geduran Si 60, Firma Merck, Darmstadt) gereinigt wird (Füllhöhe der Säule 400 mm, Durchmesser 22 mm). Eluiert wird mit einer Mischung aus 850 ml Chloroform, 150 ml Ethanol und 10 ml konzentriertem wässrigen Ammoniak.

Das nach Entfernen des Eluierungsmittels erhaltene Produkt wird mit 10 ml Ether verdünnt, eine äquivalente Menge HCl in Isopropanol zugetropft und die Mischung nach Zugabe von Impfkristallen mehrere Stunden ins Tiefkühlfach gestellt. Das auskristallisierte Hydrochlorid des 6-Chlor-2-[piperidyl-(4)-thio]-pyridins wird abgesaugt, mit Ether gewaschen und unter Ölpumpenvakuum bei 50 °C getrocknet. F. des Hydrochlorids 132–133 °C.

Das 4-Mercaptopiperidin(Hydrochlorid) kann beispielsweise ausgehend vom 1-Methyl-piperidinon-(4) auf folgendem Wege erhalten werden:

In die Lösung von 1026 g (9,066 Mol) frisch destilliertem 1-Methyl-piperidinon-(4) in 1,5 Liter Isopropanol wird unter Rühren Schwefelwasserstoff in lebhaftem Strom eingeleitet. Die Temperatur des Reaktionsgemisches wird zwischen 10–15 °C gehalten. Überschüssiger Schwefelwasserstoff wird in handelsüblicher Natriumhypochloritlösung absorbiert. Nach etwa zweistündigem Einleiten beginnt aus der Lösung das Reaktionsprodukt zu kristallisieren. Das Begasen wird für weitere 2 Stunden fortgesetzt. Das so erhaltene 1-Methyl-piperidin-4-bis(hydrosulfid)-hydrat wird abgesaugt, zweimal mit je 300 ml kaltem Isopropanol und zweimal mit je 500 ml Diethylether nachgewaschen. Die Substanz wird im Exsikkator über Phosphorpentoxid im Dunkeln aufbewahrt und sollte rasch weiter verarbeitet werden.

350 g (9,23 Mol) gepulvertes Natriumborhydrid werden in 2,5 Liter Isopropanol suspendiert. Unter Rühren werden portionsweise 1396 g (7,7 Mol) 1-Methyl-piperidin-4-bis-(hydrosulfid)-hydrat zugegeben. Die Reaktion verläuft exotherm. Es wird mit einem Eisbad gekühlt, die Temperatur sollte 25 °C nicht übersteigen. Der freiwerdende Schwefelwasserstoff wird in handelsüblicher Natriumhypochloritlösung absorbiert. Nach beendeter Zugabe wird das Kühlbad entfernt und über Nacht bei Raumtemperatur stehengelassen. Danach wird das Reaktionsgemisch etwa 60 Minuten durch kontinuierliches Steigern der Temperatur auf 80 °C erhitzt und dabei 2 Stunden belassen. Am absteigenden Kühler wird unter schwachem Vakuum (100 Torr) das Isopropanol weitgehend abdestilliert.

Der pastenförmige Rückstand wird auf Raumtemperatur abgekühlt und dann mit 1,5 Liter Diethylether versetzt. Es entsteht eine gut rührbare Suspension. Unter weiterem Kühlen werden 740 ml Eiswasser langsam zugetropft. Nachdem etwa die Hälfte der Wassermenge zugetropft ist, hat der Kolbeninhalt wieder eine schwer rührbare pastenförmige Konsistenz angenommen. Weitere Wasserzugabe führt wieder zu besserem Rühren und deutlicher Trennung zwischen organischer Phase und anorganischem Boranatrückstand. Das Rühren wird eingestellt, die Etherphase abgehebert. Der Rückstand wird 3mal mit je 500 ml frischem Ether verrührt. Die vereinigten organischen Phasen werden über Magnesiumsulfat getrocknet. Nach Filtrieren wird die Lösung unter vermindertem Druck am Rotationsverdampfer eingeengt. Der Rückstand wird einer Vakuumdestillation unterworfen. Wegen des niedrigen Siedepunktes ($Kp_2$ 35–40 °C) wird das so erhaltene 1-Methyl-4-mercapto-piperidin in einer mit Methanol/Trockeneis gekühlten Vorlage aufgefangen.

Zur Lösung von 65,5 g (0,5 Mol) 1-Methyl-4-mercapto-piperidin in 300 ml Aceton werden unter Rühren bei 15–20 °C 59,6 g (0,56 Mol) Chloräthylformiat zugetropft. Das Hydrochlorid des 1-Methyl-4-ethoxycarbonylmercapto-piperidins fällt hierbei als kristallines Produkt aus und wird nach beendeter Reaktion abgesaugt, mit Aceton gewaschen und getrocknet. Aus dem Salz wird in wässriger Lösung mit konzentrierter wässriger Ammoniaklösung die Base in Freiheit gesetzt. Die ätherische Lösung der Base wird mit $Na_2SO_4$ getrocknet, filtriert und eingeengt. Die Substanz wird durch Destillation gereinigt.

$Kp_{12}$ 128–130 °C.

Zu der auf 90 °C erhitzten Lösung von 100 g (0,48 Mol)
1-Methyl-4-ethoxycarbonylmercapto-piperidin
in 80 ml Toluol werden unter Rühren 106,3 g (0,88 Mol) Chloräthylformiat im Laufe von 30 Minuten zugetropft. Danach wird 2 Stunden auf 100–110 °C erhitzt. Nach abermaligem Zufügen von 40 g Chloräthylformiat wird noch weitere 3 Stunden erhitzt. Nach Stehen über Nacht bei Raumtemperatur wird über ein Glasfaserfilter abgesaugt. Die Lösung wird am Rotationsverdampfer eingeengt, der Rückstand destilliert. Man erhält 120 g (= 94% d. Theorie) 1-Ethoxycarbonyl-4-ethoxycarbonylmercapto-piperidin.

$Kp_{0,2}$ 138–140 °C.

269,7 g (1,032 Mol) 1-Ethoxycarbonyl-4-ethoxycarbonylmercapto-piperidin werden in einem Gemisch aus 886 ml (10,3 Mol) konzentrierter wässriger Salzsäure und 443 ml Eisessig gelöst. Unter Rühren wird im Laufe von 1 Stunde auf Rückflusstemperatur erhitzt.

Nach 60 stündiger Reaktionszeit wird die Lösung am Rotationsverdampfer eingeengt.

Um das Restlösungsmittelgemisch weitgehend zu entfernen, wird der kristalline Rückstand 2 mal mit je 200 ml Isopropanol versetzt. Danach wird wieder abdestilliert. Das so erhaltene 4-Mercapto-piperidin-Hydrochlorid wird aus Äthanol umkristallisiert.

F. 183–184 °C (Zersetzung.

Die Ausbeute beträgt 117,6 g und erhöht sich nach Aufarbeiten der Mutterlauge um weitere 27,5 g; das sind ~96% der Theorie.

Beispiel 23

6-Chlor-2-[N-Methyl-piperidyl-(4)-thio]-pyridin-N-oxid

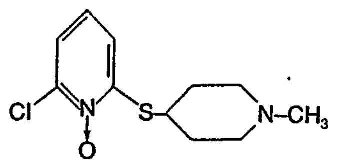

4,9 g (0,03 Mol) 2,6-Dichlor-pyridin-N-oxid werden zu einer Lösung aus 4,5 g (0,035 Mol) N-Methyl-4-mercapto-piperidin in 20 ml Ethanol eingetropft. Die Piperidin-Verbindung liegt als Natriumsalz vor und wurde mittels 11,9 g (0,035 Mol) 20%iger Natriumethylatlösung zuvor hergestellt. Das Reaktionsgemisch wird auf 50 °C erwärmt und 3 Stunden bei dieser Temperatur belassen. Danach wird das Reaktionsgemisch in ca. 200 ml Eiswasser gegossen, wobei eine kristalline Substanz ausfällt. Es wird abgesaugt mehrmals mit Wasser nachgewaschen, getrocknet und das Ethanol umkristallisiert. F. 129–130 °C.

Die Ausgangssubstanz 2,6-Dichlor-pyridin-N-oxid wird beispielsweise wie folgt erhalten: Eine Lösung aus 16 g (0,108 Mol) 2,6-Dichlor-pyridin und 17 g 35%iges Perhydrol (entsprechend 5,9 g aktivem $H_2O_2$, ungefähr 0,17 Mol) und 250 g Trifluoressigsäure wird 8 Stunden lang auf dem Wassserbad erhitzt. Innentemperatur ca. 75 °C. Danach wird die Lösung in 1,5 Liter Wasser eingegossen. Es scheidet sich hierbei eine geringe Menge eines kristallinen Produktes aus, welches unverändertes 2,6-Dichlorpyridin ist. Nach Absaugen desselben wird die Lösung im Wasserstrahl vakuum bei einer Badtemperatur von 30–35 °C weitgehend eingeengt. Der flüssige Rückstand wird in 500 ml Chloroform gelöst und unter Rühren wird soviel wasserfreie Pottasche zugegeben, bis keine Gasentwicklung mehr stattfindet und darüberhinaus das Wasser gebunden wird. Man filtriert ab bei einer Badtemperatur von 30–35 °C und engt in schwachem Vakuum zur Trockne ein. 2,6-Dichlor-pyridin-N-oxid wird als kristalliner Rückstand erhalten.
F. 137–138 °C.

Beispiel 24
Herstellung aus einer Verbindung II, worin Z SH oder OH ist und einer Verbindung III, worin Y Halogen ist:

Ein Gemisch aus 0,06 Mol einer Verbindung der Formel III, worin Y Halogen ist und 0,06 Mol 6-Chlor-2-mercaptopyridin-Natriumsalz in beispielsweise 60 ml n-Propanol wird mehrere Stunden (zum Beispiel 6 Stunden) unter Rühren und Rückfluss erhitzt. Nach dem Abkühlen wird von unlöslichen Rückständen abgesaugt. Die Lösung wird eingeengt und der sirupöse Rückstand mit isopropanolischer Salzsäure angesäuert. Nach dem Verdünnen mit Aceton kristallisiert das Hydrochlorid aus. Die Kristalle werden isoliert, mit Aceton und anschliessend mit Wasser gewaschen. Die weitere Reinigung erfolgt durch Umkristallisation.

Aus 9,3 g N-Methyl-2-(2-chlor-ethyl)-piperidin und 9,6 g 2-Natriummercapto-6-chlor-pyridin wird auf die angegebene Weise das 2-[2-(N-Methyl-piperidyl-(2)-ethylmercapto]-6-chlor-pyridin der folgenden Formel erhalten

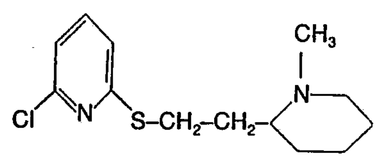

F. des Hydrochlorids: 165–167 °C; die Umkristallisation erfolgte aus Ethanol/Ether.

Die Verbindungen gemäss den Beispielen 13–15 von Tabelle 1 wurden zusätzlich auch auf diese Weise hergestellt.

Das 6-Chlor-2-mercapto-pyridin kann beispielsweise wie folgt hergestellt werden:

Zu 700 ml n-Butanol werden 103,1 g (0,70 Mol) 2,6-Dichlorpyridin und 110,0 g (2 × 0,70 Mol) Natriumhydrogensulfid x $H_2O$ (71%ig) gegeben und insgesamt 10 Stunden unter Rückfluss erhitzt. Bei 35 °C wird abgesaugt und das Filtrat im Vakuum bei 60 °C eingedampft (170 g Rückstand). Der Rückstand wird mit 1 Liter Ether verrührt und über Nacht stehen gelassen. Das gebildete feste Produkt (Natriumsalz) wird abgesaugt, gut mit Ether gewaschen und 24 Stunden im Vakuum bei 35 °C getrocknet.

Die Beispiele 25–44 (Tabelle 2) betreffen die Einführung des Restes $R_3$ in Verbindungen der Formel I, worin $R_3$ Wasserstoff ist durch Alkylierung oder Acylierung.

Allgemeine Vorschrift für die Beispiele 25–44

Das Amin der Formel I, worin $R_3$ Wasserstoff ist, wird mit dem Halogenid der Formel $HalR_3$ (Überschuss des Halogenids von 10–300 Mol%) und der Base (2–6fachem Überschuss) im Lösungsmittel über mehrere Stunden (bis nach dünnschichtchromatografischer Kontrolle keine weitere Reaktion mehr zu verzeichnen ist) unter Rückfluss erhitzt. Nach Abkühlen, Abfiltrieren des Niederschlages und Einengen des Lösungsmittels erfolgt die weitere Aufarbeitung in üblicher Weise durch Salzbildung, in einigen Fällen ist eine chromatografische Reinigung an Kieselgel erforderlich.

Die so hergestellten Verbindungen der Formel

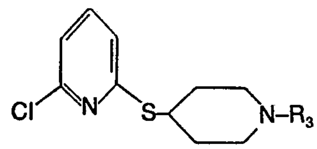

sind in der Tabelle 2 aufgeführt.

Tabelle 2

| Beispiel Nr. | $R_3$ | Lösungsmittel | basische Verbindung | Salz | F. |
|---|---|---|---|---|---|
| 25 | $(CH_2)_2$– 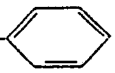 | Dioxan | TEA | Maleat | 126–127°C |
| 26 | $CH_2CH=CH_2$ | Dioxan | TEA | Oxalat | 134–136°C |
| 27 | $CH_2CH_2OH$ | Xylol | $K_2CO_3$ | Base | 67–70°C |
| 28 | $CH_2CH_2CO$– 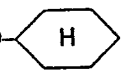 | Dioxan | TEA | HCl | 169–173°C |
| 29 | $C_3H_7$ | Dioxan | TEA | HCl | 172–175°C |
| 30 | $CH_2$– 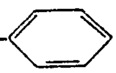 | Dioxan | TEA | Oxalat | 175–178°C |
| 31 | n-$C_6H_{13}$ | DMAC/Toluol | $NaHCO_3$ | Oxalat | 155–156°C |
| 32 | $C_2H_5$ | Dioxan | TEA | HCl | 203–205°C |
| 33 | $CH\ (CH_3)_2$ | Dioxan | TEA | Oxalat | 160°C |
| 34 | $CH_2CH(CH_3)–CH_3$ | DMAC/Toluol | $NaHCO_3$ | Maleat | 135–136°C |
| 35 | $CH_2CH_2CH_2F$ | DMAC/Toluol | $NaHCO_3$ | HCl | 167–169°C |
| 36 | $CH_2CH_2CH_2N(CH_3)_2$ | DMAC/Toluol | $NaHCO_3$ | HCl | 273–277°C |
| 37 | 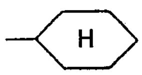 | DMAC/Toluol | $NaHCO_3/K_2CO_3$ | HCl | 205–206°C |
| 38 | $COCH_3$ | Dioxan | TEA | Base | 210–215°C |
| 39 | $COCH_2CH_2$– 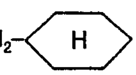 | Dioxan | $K_2CO_3$ | Base | $R_f$-Wert: 0,79<br>Laufmittel: Chloroform/Methanol/ 25%iges $NH_3$ (95:4:1) |
| 40 | $CH_2$– 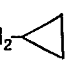 | DMAC | $K_2CO_3$ | HCL | 173–175°C |
| 41 | $(CH_2)_2CH$ 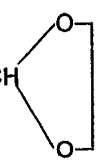 | Toluol | $NaHCO_3/K_2CO_3$ | Oxalat | 176–178°C |
| 42 | $(CH_2)_3$– 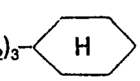 | Dioxan | TEA | HCl | 198–201°C |
| 43 | $(CH_2)_2–O–CH_3$ | DMAC/Toluol | $NaHCO_3/K_2CO_3$ | Base | $R_f$-Wert: 0,50<br>Laufmittel: Chloroform/Methanol/ 25%iges $HN_3$ (95:4:1) |
| 44 | 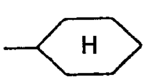 | Toluol | $NaHCO_3$ | HCl | 208–210°C |

TEA = Triethylamin
DMAC = Dimethylacetamid

Beispiel 45
2-[N-(2,3-Dihydroxy-propyl)-piperidyl-(4)-thio]-6-chlor-pyridin

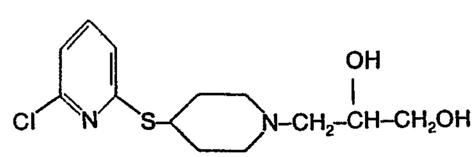

4,85 g (0,0212 Mol) 2-(Piperidyl-(4)-thio)-6-chlor-pyridin (freie Base) werden zusammen mit 1,4 ml Glycid und 40 ml Isopropanol 5 Stunden zum Sieden erhitzt. Die isopropanolische Lösung wird eingeengt und mit 6 ml isopropanolischer HCl versetzt. Das Hydrochlorid kristallisiert langsam aus. F. des Hydrochlorids: 115–121 °C.

Beispiel 46
2-[N-Methyl-piperidyl-(4)-oxy]-3-acetylamino-6-(4-fluorbenzylamino)-pyridin

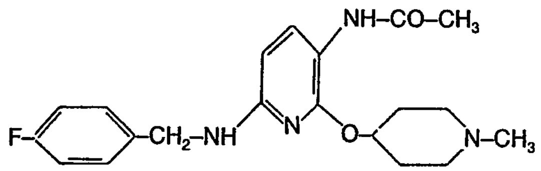

Zu der Hydrierlösung von 0,025 Mol
2-[N-Methyl-piperidyl-(4)-oxy]-3-amino-6-(4-fluor-benzylamino)-pyridin,
die durch Hydrierung von 4,5 g (0,025 Mol) der entsprechenden 3-Nitro-Verbindung in 125 ml Dioxan in Gegenwart eines Palladium-Kohle-Katalysators bei 60 °C und 5 bar erhalten wurde, werden unter Stickstoff 1,8 ml Acetylchlorid hinzugefügt. Das ausfallende Hydrochlorid wird abgenutscht und mit Natronlauge die Base in herkömmlicher Weise freigesetzt.
F. des Hydrochlorids: 188–190 °C.

Beispiel 47
2-[N-Methyl-piperidyl-(4)-oxy]-3-ethoxycarbonylamino-6-(4-fluor-benzylamino)-pyridin

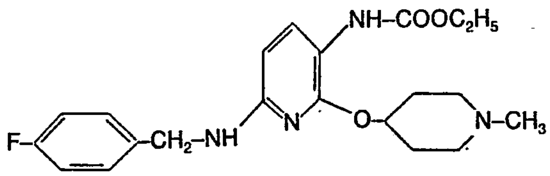

Zu der Hydrierlösung von 0,02 Mol
2-[N-Methyl-piperidyl-(4)-oxy]-3-amino-6-(4-fluor-benzylamino)-pyridin,
die durch Hydrierung von 7,2 g (0,02 Mol) der entsprechenden 3-Nitro-Verbindung in 125 ml Dioxan in Gegenwart eines Palladium-Kohle-Katalysators bei 60 °C und 5 bar erhalten wurde, werden unter Rühren und Stickstoffatmosphäre 4 ml Chlorameisensäureethylester getropft. Es wird ½ Stunde bei Raumtemperatur gerührt, die Lösung eingeengt und der Rückstand mit einem Benzin-Ether-Gemisch 1:1 ausgerührt. Die auskristallisierte Substanz wird abgesaugt und aus Methanol umkristallisiert.
F. des Dihydrochlorids: 202–207 °C.

Die freie Base erhält man aus dem Dihydrochlorid beispielsweise durch Behandeln mit Natronlauge.
F. der Base: 163–169 °C (ohne Umkristallisation).

Beispiele 48–54 (Tabelle 3)

Diese Beispiele betreffen den Austausch einer Methylgruppe an dem Piperidinring von Verbindungen der Formel I ($R_3 = CH_3$) gegen die Ethoxycarbonylgruppe und die anschliessende Abspaltung der letzteren. Allgemeine Vorschrift:

0,09 Mol einer Verbindung der Formel I, worin $R_3 = CH_3$ ist, werden in 30 ml Toluol gelöst und unter Rühren zu einer auf 85 °C erwärmten Lösung von 0,18 Mol Chlorameisensäureethylester in 30 ml Toluol im Laufe von ca. 30 Minuten zugetropft. Nach beendetem Zutropfen wird noch 6 Stunden unter Rühren und Rückfluss erhitzt, nach dem Abkühlen von festen Bestandteilen abfiltriert und die Lösung zur Trockene eingeengt. Das resultierende N-Carbethoxy-produkt wird in der Regel nicht weiter gereinigt und als Rohprodukt eingesetzt. Das Rohprodukt (Verbindung der Formel I, worin $R_3 = CO\text{-}OC_2H_5$ ist), wird in einem Gemisch aus 80 g konzentrierter wässriger Salzsäure und 40 ml Eisessig gelöst. Es wird 15 Stunden unter Rückfluss erhitzt. Danach wird zur Trockene eingeengt, der Rückstand mit Isopropanol versetzt und erneut eingeengt. Der feste Rückstand wird durch Umkristallisation gereinigt. Die hergestellten Verbindungen der folgenden Formel

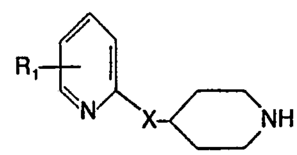

sind in der Tabelle 3 aufgeführt.

Tabelle 3

| Beispiel Nr. | $R_1$ | X | Salz | F. |
|---|---|---|---|---|
| 45 | 6–Cl | 0 | HCl | 219–220° C |
| 49 | 6–Cl | S | Maleat | 144–145° C |
| 50 | 6–Cl | S | HCl | 132–133° C |
| 51 | H | S | 2HCl | 256–257° C |
| 52 | 6–$CH_3$ | S | 2HCl | 243–244° C |
| 53 | 5–Cl | S | HCl | |
| 54 | 3–Cl | S | HCl | 201–202° C |

Beispiel 55
(Austausch einer Methylgruppe des Piperidinringes gegen Ethoxycarbonyl und Abspaltung der letzteren)
6-Chlor-2-[piperidyl-(4)-thio]-pyridin-N-oxid

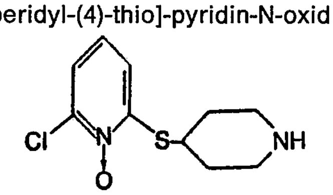

Die Lösung von 3,5 g
2-[1-Methylpiperidin-4-mercapto]-6-chlor-pyridin-N-oxid
in 20 ml Chlorameisensäureethylester wird unter Rühren und Rückfluss erhitzt. Nach jeweils 3 Stunden werden weitere 20 ml Chlorameisensäureethylester zugefügt (insgesamt 3 mal). Insgesamt wird also 9 Stunden erhitzt. Danach wird zur Trockne eingeengt. Der feste Rückstand wird aus Ethanol umkristallisiert.

Das so erhaltene 6-Chlor-2-[N-carbethoxy-piperidyl-(4)-thio]-pyridin schmilzt bei 151–152 °C.

2,4 g (0,0075 Mol) dieser Carbethoxy-Verbindung werden mit 7,6 g konzentrierter wässriger HCl (0,075 Mol) und 5 ml Eisessig 16 Stunden unter Rühren und Rückfluss erhitzt. Danach wird die Lösung eingeengt und der kristalline Rückstand mit 25 ml Methanol versetzt. Es wird erneut zur Trockne eingeengt. Danach wird der Rückstand in der benötigten Menge Methanol in der Siedehitze gelöst. Nach Zufügen von Kieselgur wird filtriert und mit Ether bis zur beginnenden Trübung versetzt. Das
6-Chlor-2-[piperidyl-(4)-thio]-pyridin-N-oxid-hydrochlorid
kristallisiert aus. Nach einstündigem Stehen im Eisbad wird abgesaugt, mit Aceton gewaschen und getrocknet.
F. des Hydrochlorids 232–233 (Zersetzung).

Beispiel 56
2-[N-Methyl-piperidyl-(4)-oxy]-3-nitro-6-(4-fluorbenzylamino)-pyridin

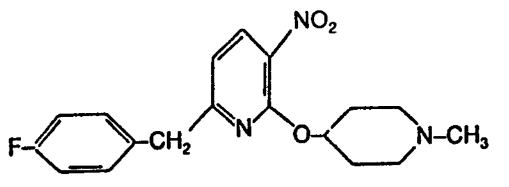

31 g (0,114 Mol)
2-[N-Methyl-piperidyl-(4)-oxy)-3-nitro-6-chlor-pyridin,
15,6 g (0,125 Mol) 4-Fluor-benzylamin, 34,5 ml (0,125 Mol) Triethylamin und 70 ml Isopropanol werden 7 Stunden unter Rückfluss erhitzt. Das nach dem Abkühlen anfallende Triethylammoniumchlorid wird abgetrennt und die Mutterlauge im Vakuum eingeengt. Dabei kristallisiert die Titelverbindung in Form der freien Base aus und wird abgenutscht und getrocknet.
F.: 90–94 °C.

Beispiel 57
2-[N-Methyl-piperidyl-(4)-oxy]-3-amino-6-(4-fluorbenzylamino)-pyridin

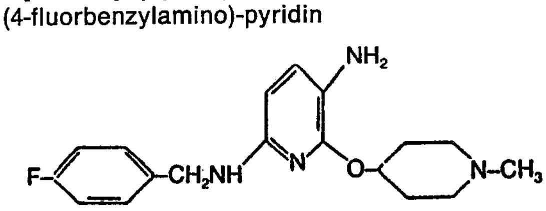

4,5 g (0,0125 Mol)
2-[N-Methyl-piperidyl-(4)-oxy]-3-nitro-6-(4-fluor-benzylamino)-pyridin
und 0,6 g Palladium auf Aktivkohle (5%) werden in 125 ml Dioxan suspendiert und 5 Stunden bei 60 °C und 5 bar in einer Hydrier-Apparatur hydriert. Nach dem Entfernen des Katalysators wird mit überschüssiger isopropanolischer HCl versetzt. Das ausfallende Dihydrochlorid wird abgenutscht und aus Ethanol unter Zusatz von wenig Ether umkristallisiert.
F. des Dihydrochlorids: 245–248 °C.

Beispiel 58
2-[N-Methyl-piperidyl-(4)-thio]-6-chlor-pyridin-sulfoxid und -sulfon
5 g (0,018 Mol)
2-[N-Methyl-piperidyl-(4)-thio]-6-chlor-pyridin-Hydrochlorid
werden in 50 ml Methanol gelöst. Mit 1 n wässriger Salzsäure wird auf pH 4 eingestellt und die Lösung auf 50 °C erwärmt. Unter Rühren werden 2,4 g (ca. 0,021 Mol) 30%iges $H_2O_2$ zugetropft. Das Reaktionsgemisch wird zum Sieden erhitzt. Nach ca. 2 Stunden werden erneut ca. 2,5 g 30%iges $H_2O_2$ zugefügt. Nach insgesamt 16stündiger Reaktionszeit wird überschüssiges $H_2O_2$ durch Zugabe von konzentrierter Ameisensäure vernichtet. Die Lösung wird bei Raumtemperatur eingeengt und der sirupöse Rückstand in wenig Wasser gelöst. Mit konzentrierter Natronlauge wird alkalisch gemacht und die freie Base durch mehrmalige Extraktion mit Ether isoliert. Nach Trocknen der organischen Phase wird das Lösungsmittel im Vakuum abdestilliert, der kristalline Rückstand wird durch Säulenchromatographie an Kieselgel (Elutionsmittel $CHCl_3$/Methanol/Ammoniak 90:9:1) gereinigt. Es werden zwei Substanzen isoliert:
1. 400 mg Sulfon F.: 123–124 °C
2. 2,3 g Sulfoxid F.: 136–137 °C.

Das Sulfon kann auf folgendem Weg zum Beispiel in grösserer Ausbeute erhalten werden:
3 g (0,012 Mol)
2-[N-Methyl-piperidyl-(4)-thio]-6-chlor-pyridin
werden in 30 ml Eisessig gelöst. Unter Rühren tropft man bei 40 °C eine Lösung von 3,5 g (0,022 Mol) Kaliumpermanganat in 50 ml Wasser zu (innerhalb von 60 Minuten). Nach beendetem Zutropfen wird für 2 Stunden auf 60 °C erhitzt. Der gebildete Niederschlag wird abfiltriert und die Lösung zur Trockene eingeengt. Der resultierende kristalline Rückstand wird mit Ether verrührt und abgesaugt.
F.: 124–125 °C.

Beispiele für pharmazeutische Zubereitungen

Beispiel 1: Kapseln
50 g Wirksubstanz werden mit 350 g mikrokristalliner Cellulose, 590 g Milchzucker und 10 g Magnesiumstearat gemischt.
Die Mischung wird in einer Menge von jeweils 100 mg in Hartgelatine-Steckkapseln der Grösse 3 gefüllt.
Eine Kapsel enthält 5 mg Wirksubstanz.

Beispiel 2: Ampullen
10 g Wirksubstanz werden zusammen mit 30,48 g Natriumchlorid in etwa 3,8 Litern Wasser für Injektionszwecke gelöst. Die erhaltene Lösung wird mit 0,1 N Natronlauge auf pH 7,4 eingestellt und mit Wasser für Injektionszwecke auf 4 Liter aufgefüllt. Die Lösung wird durch ein Membranfilter geeigneter Porenweite steril filtriert. Das Filtrat wird unter aseptischen Bedingungen zu 2 ml in Ampullen abgefüllt. Die Ampullen werden anschliessend 20 Minuten lang im gepannten Wasserdampf bei 121 °C sterilisiert.

Eine Ampulle enthält 5 mg Wirksubstanz in 2 ml Lösung.

**Patentansprüche**

1. Verbindungen der Formel

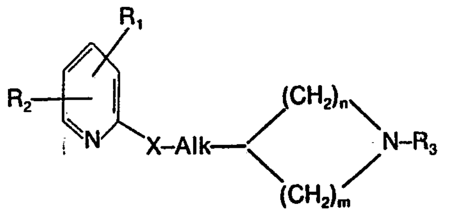

I

worin die Reste $R_1$ und $R_2$ gleich oder verschieden sind und Wasserstoff, Halogenatome, eine Trifluormethylgruppe, eine Cyanogruppe, eine Nitrogruppe, eine Aminogruppe, eine Mono-$C_1$–$C_6$-alkylaminogruppe, eine Di-$C_1$–$C_6$-alkylaminogruppe, eine Aminogruppe, die durch einen Phenylrest, einen Mono- oder Dihalogenphenylrest oder einen Phenyl-$C_1$–$C_4$-alkylrest substituiert ist, eine $C_1$–$C_6$-Alkanoylaminogruppe, eine $C_1$–$C_6$-Alkoxycarbonylaminogruppe, eine gegebenenfalls durch einen Phenylrest substituierte $C_1$–$C_6$-Alkylgruppe, eine Phenylgruppe, eine Hydroxygruppe, eine $C_1$–$C_6$-Alkoxygruppe, eine Phenoxygruppe, eine Carboxygruppe, eine Carb-$C_1$–$C_6$-alkoxygruppe oder eine gegebenenfalls durch eine oder zwei $C_1$–$C_6$-Alkylgruppen substituierte Carbamoylgruppe bedeuten, der Rest $R_3$ Wasserstoff, eine $C_1$–$C_6$-Alkylgruppe, eine $C_3$–$C_6$-Alkenylgruppe, eine $C_3$–$C_6$-Alkinylgruppe, eine $C_3$–$C_7$-Cycloalkylgruppe, eine $C_5$–$C_7$-Cycloalkenylgruppe, eine Phenyl-$C_1$–$C_4$-alkylgruppe, eine Carb-$C_1$–$C_6$-alkoxygruppe, eine gegebenenfalls durch einen $C_3$–$C_6$-Cycloalkylrest substituierte $C_2$–$C_6$-Alkanoylgruppe oder eine $C_1$–$C_4$-Alkylgruppe ist, die am selben C-Atom zwei $C_1$–$C_6$-Alkoxygruppen oder eine $C_2$–$C_4$-Alkylendioxygruppe enthält oder worin $R_3$ eine $C_1$–$C_6$-Alkylgruppe bedeutet, die ein- oder zweifach durch $C_3$–$C_7$-Cycloalkylgruppen, Hydroxygruppen, $C_1$–$C_6$-Alkoxygruppen, Halogenatome, Sulfogruppen ($-SO_3H$), Aminogruppen, $C_1$–$C_6$-Alkylaminogruppen, Di-$C_1$–$C_6$-alkylaminogruppen, $C_1$–$C_6$-Alkylcarbonylgruppen, $C_3$–$C_7$-Cycloalkylcarbonylgruppen, Carb-$C_1$–$C_6$-alkoxygruppen oder Benzoylgruppen substituiert ist, X Sauerstoff, Schwefel, SO oder $SO_2$ bedeutet, Alk Alkylen mit 0–4 C-Atomen ist und n und m gleich oder verschieden sind und die Zahlen 1–3 annehmen können, wobei n auch 0 sein kann, wenn Alk Alkylen mit mindestens einem Kohlenstoffatom ist und m für diesen Fall die Zahlen 2–6 annimmt und wobei die Gruppierung

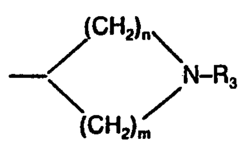

auch den Chinuclidylrest oder den Tropanylrest darstellen kann, deren Pyridin-N-oxide und deren pharmazeutisch verwendbare Salze, ausgenommen die Verbindungen 4-(2-Methyl-6-pyridyloxymethyl)-piperidin und 4-(2-Pyridyl-oxymethyl)-piperidin.

2. Verbindungen der Formel gemäss Anspruch 1, worin $R_1$ Wasserstoff, Amino oder $C_2$–$C_6$-Alkanoylamino, $R_2$ Chlor, Brom, Fluor, $C_1$–$C_4$-Alkyl oder $C_1$–$C_4$-Alkoxy, X Schwefel und $R_3$ Wasserstoff, $C_3$–$C_6$-Alkenyl oder $C_1$–$C_6$-Alkyl, welches auch ein Halogenatom, eine Methylendioxygruppe oder eine oder zwei Hydroxygruppen enthalten kann, bedeutet, und worin der basisch gesättigte Ring

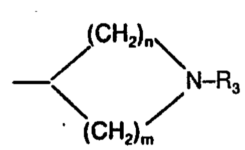

einen Pyrrolidylrest, einen Piperidylrest oder einen Homopiperidylrest darstellt, welcher jeweils am N-Atom des Rest $R_3$ enthält, und deren Salze.

3. Verbindungen der Formel gemäss Anspruch 1, worin $R_1$ Wasserstoff, $R_2$ Chlor, Brom oder Fluor, X Schwefel und $R_3$ Wasserstoff oder eine $C_1$–$C_6$-Alkylgruppe bedeutet, und der basische Ring

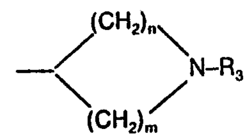

einen Pyrrolidylrest, einen Piperidylrest oder einen Homopiperidylrest darstellt, welcher jeweils am N-Atom den Rest $R_3$ enthält, und deren Salze.

4. Verfahren zur Herstellung von Verbindungen gemäss Anspruch 1, worin die Reste $R_1$ und $R_2$ gleich oder verschieden sind und Wasserstoff, Halogenatome, eine Trifluormethylgruppe, eine Cyangruppe, eine Nitrogruppe, eine Aminogruppe, eine Mono-$C_1$–$C_6$-alkylaminogruppe, eine Di-$C_1$–$C_6$-alkylaminogruppe, eine Aminogruppe, die durch einen Phenylrest, einen Mono- oder Dihalogenphenylrest oder einen Phenyl-$C_1$–$C_4$-alkylrest substituiert ist, eine $C_1$–$C_6$-Alkanoylaminogruppe, eine $C_1$–$C_6$-Alkoxycarbonylaminogruppe, eine gegebenenfalls durch einen Phenylrest substituierte $C_1$–$C_6$-Alkylgruppe, eine Phenylgruppe, eine Hydroxygruppe, eine $C_1$–$C_6$-Alkoxygruppe, eine Phenoxygruppe, eine Carboxygruppe, eine Carb-$C_1$–$C_6$-alkoxygruppe oder eine gegebenenfalls durch eine oder zwei $C_1$–$C_6$-Alkylgruppen substituierte Carbamoylgruppe bedeuten, der Rest $R_3$ Wasserstoff, eine $C_1$–$C_6$-Alkylgruppe, eine $C_3$–$C_6$-Alkenylgruppe, eine $C_3$–$C_6$-Alkinylgruppe, eine $C_3$–$C_7$-Cycloalkylgruppe, eine $C_5$–$C_7$-Cycloalkenylgruppe, eine Phenyl-$C_1$–$C_4$-alkylgruppe, eine Carb-$C_1$–$C_6$-alkoxygruppe, eine gegebenenfalls durch einen $C_3$–$C_6$-Cycloalkylrest substituierte $C_2$–$C_6$-Alkanoylgruppe oder eine $C_1$–$C_4$-Alkylgruppe ist, die am selben C-Atom zwei $C_1$–$C_6$-Alkoxygruppen oder eine $C_2$–$C_4$-Alkylendioxygruppe enthält oder worin $R_3$ eine $C_1$–$C_6$-Alkylgruppe bedeutet, die ein- oder zweifach durch $C_3$–$C_7$-Cycloalkylgruppen, Hydroxygruppen, $C_1$–$C_6$-Alkoxygruppen, Halogenatome, Sulfogruppen ($-SO_3H$), Aminogruppen, $C_1$–$C_6$-Alkylaminogruppen, Di-$C_1$–$C_6$-alkylaminogruppen, $C_1$–$C_6$-Alkylcarbonylgruppen, $C_3$–$C_7$-Cycloalkylcarbonylgruppen, Carb-$C_1$–$C_6$-alkoxygruppen oder Benzoylgruppen substituiert

ist, X Sauerstoff, Schwefel, SO oder $SO_2$ bedeutet, Alk Alkylen mit 0–4 C-Atomen ist und n und m gleich oder verschieden sind und die Zahlen 1–3 annehmen können, wobei n auch 0 sein kann, wenn Alk Alkylen mit mindestens einem Kohlenstoffatom ist und m für diesen Fall die Zahlen 2–6 annimmt und wobei die Gruppierung

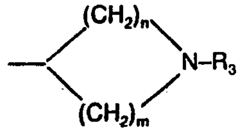

auch den Chinuclidylrest oder den Tropanylrest darstellen kann, sowie deren Pyridin-N-oxiden dadurch gekennzeichnet, dass man eine Verbindung der allgemeinen Formel

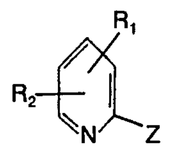

II

oder deren Pyridin-N-oxid, worin $R_1$ und $R_2$ die angegebenen Bedeutungen haben mit einer Verbindung der allgemeinen Formel

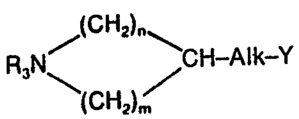

III

umsetzt, wobei in der Formel III $R_3$, n und m und Alk die angegebenen Bedeutungen haben, $R_3$ ausserdem auch die Gruppe S sein kann und S eine übliche Amino-Schutzgruppe ist und Y ein Halogenatom, eine $C_1$–$C_6$-Alkyl-sulfonyloxygruppe oder eine Aryl-sulfonyloxygruppe ist, falls Z der Formel II eine Hydroxygruppe oder eine Mercaptogruppe ist, oder worin Y eine Hydroxygruppe oder eine Mercaptogruppe bedeutet, falls Z der Formel II ein Halogenatom ist, und eine vorhandene Gruppe S abspaltet und/oder in Verbindungen der Formel I die Reste $R_1$, $R_2$ und $R_3$ in andere hierfür mögliche Bedeutungen überführt und/oder Verbindungen der Formel I, worin die Reste $R_1$–$R_3$ sowie Alk, X, n und m die angegebenen Bedeutungen haben in die entsprechenden Sulfone, Sulfoxide oder Pyridin-N-oxide überführt.

5. Verfahren nach Anspruch 2, dadurch gekennzeichnet, dass man die erhaltenen Verbindungen in ihre Salze überführt.

6. Arzneimittel, dadurch gekennzeichnet, dass es als Wirkstoff eine Verbindung nach einem oder mehreren der vorangegangenen Ansprüche zusammen mit einem üblichen pharmazeutischen Träger und/oder einem Verdünnungsmittel enthält.

7. Verfahren zur Herstellung eines Arzneimittels, dadurch gekennzeichnet, dass eine Verbindung nach einem oder mehreren der vorangegangenen Ansprüche mit gebräuchlichen pharmazeutischen Trägerstoffen beziehungsweise Verdünnungsmitteln zu pharmazeutischen Zubereitungen verarbeitet wird.

8. Verwendung von Verbindungen nach einem oder mehreren der vorangegangenen Ansprüche zur Herstellung eines Arzneimittels.

**Claims**

1. Compounds corresponding to the following formula

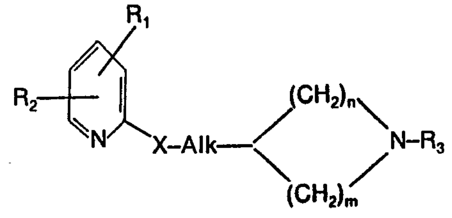

I

in which the substituents $R_1$ and $R_2$ may be the same or different and represent hydrogen, halogen atoms, a trifluoromethyl group, a cyano group, a nitro group, an amino group, a mono-$C_1$–$C_6$-alkylamino group, a di-$C_1$–$C_6$-alkylamino group, an amino group substituted by a phenyl radical, a mono- or dihalophenyl radical or a phenyl-$C_1$–$C_4$-alkyl radical, a $C_1$–$C_6$-alkanoylamino group, a $C_1$–$C_6$-alkoxycarbonylamino group, an optionally phenyl-substituted $C_1$–$C_6$ alkyl group, a phenyl group, a hydroxy group, a $C_1$–$C_6$ alkoxy group, a phenoxy group, a carboxy group, a carb-$C_1$–$C_6$-alkoxy group or a carbamoyl group optionally substituted by one or two $C_1$–$C_6$ alkyl groups; $R_3$ is hydrogen, a $C_1$–$C_6$ alkyl group, a $C_3$–$C_6$ alkenyl group, a $C_3$–$C_6$ alkynyl group, a $C_3$–$C_7$ cycloalkyl group, a $C_5$–$C_7$ cycloalkenyl group, a phenyl-$C_1$–$C_4$-alkyl group, a carb-$C_1$–$C_6$-alkoxy group, a $C_2$–$C_6$ alkanoyl group optionally substituted by a $C_3$–$C_6$ cycloalkyl radical or a $C_1$–$C_4$ alkyl group which contains two $C_1$–$C_6$ alkoxy groups or one $C_2$–$C_4$ alkylendioxy group at the same C atom, or $R_3$ is a $C_1$–$C_6$ alkyl group substituted once or twice by $C_3$–$C_7$ cycloalkyl groups, hydroxy groups, $C_1$–$C_6$ alkoxy groups, halogen atoms, sulfo groups ($-SO_3H$), amino groups, $C_1$–$C_6$ alkylamino groups, di-$C_1$–$C_6$-alkylamino groups, $C_1$–$C_6$ alkylcarbonyl groups, $C_3$–$C_7$ cycloalkylcarbonyl groups, carb-$C_1$–$C_6$-alkoxy groups or benzoyl groups, X is oxygen, sulfur, SO or $SO_2$, Alk is $C_0$–$C_4$ alkylene and n and m may be the same or different and may assume values of 1 to 3, although n may also be 0 where Alk is alkylene containing at least one carbon atom, in which case m may assume a value of 2 to 6, and the group

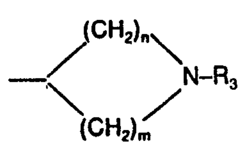

may also represent the quinuclidyl radical or the tropanyl radical, pyridine-N-oxides thereof and pharmaceutically useable salts thereof except for the compounds 4-(2-methyl-6-pyridyloxymethyl)-piperidine and 4-(2-pyridyloxymethyl)-piperidine.

2. Compounds corresponding to the formula in claim 1, in which $R_1$ is hydrogen, amino or $C_2$–$C_6$ alkanoylamino, $R_2$ is chlorine, bromine, fluorine,

$C_1$–$C_4$ alkyl or $C_1$–$C_4$ alkoxy, X is sulfur and $R_3$ is hydrogen, $C_3$–$C_6$ alkenyl or $C_1$–$C_6$ alkyl, which may also contain a halogen atom, a methylenedioxy group or one or two hydroxy groups, and in which the base-saturated ring

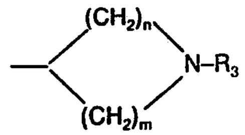

is a pyrrolidyl radical, a piperidyl radical or a homopiperidyl radical containing the substituent $R_3$ at the nitrogen atom, and salts thereof.

3. Compounds corresponding to the formula in claim 1, in which $R_1$ is hydrogen, $R_2$ is chlorine, bromine or fluorine, X is sulfur and $R_3$ is hydrogen or a $C_1$–$C_6$ alkyl group and the basic ring

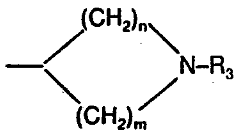

is a pyrrolidyl, a piperidyl or a homopiperidyl radical containing the substituent $R_3$ at the nitrogen atom, and salts thereof.

4. A process for the preparation of the compounds claimed in claim 1, in which the substituents $R_1$ and $R_2$ may be the same or different and represent hydrogen, halogen atoms, a trifluoromethyl group, a cyano group, a nitro group, an amino group, a mono-$C_1$–$C_6$-alkylamino group, a di-$C_1$–$C_6$-alkylamino group, an amino group substituted by a phenyl radical, a mono- or dihalophenyl radical or a phenyl-$C_1$–$C_4$-alkyl radical, a $C_1$–$C_6$ alkanoylamino group, a $C_1$–$C_6$-alkoxycarbonylamino group, an optionally phenyl-substituted $C_1$–$C_6$ alkyl group, a phenyl group, a hydroxy group, a $C_1$–$C_6$ alkoxy group, a phenoxy group, a carboxy group, a carb-$C_1$–$C_6$-alkoxy group or a carbamoyl group optionally substituted by one or two $C_1$–$C_6$ alkyl groups; $R_3$ is hydrogen, a $C_1$–$C_6$ alkyl group, a $C_3$–$C_6$ alkenyl group, a $C_3$–$C_6$ alkynyl group, a $C_3$–$C_7$ cycloalkyl group, a $C_5$–$C_7$ cycloalkenyl group, a phenyl-$C_1$–$C_4$-alkyl group, a carb-$C_1$–$C_6$-alkoxy group, a $C_2$–$C_6$ alkanoyl group optionally substituted by a $C_3$–$C_6$ cycloalkyl radical or a $C_1$–$C_4$ alkyl group which contains two $C_1$–$C_6$ alkoxy groups or one $C_2$–$C_4$ alkylenedioxy group at the same C atom, or $R_3$ is a $C_1$–$C_6$ alkyl group substituted once or twice by $C_3$–$C_7$ cycloalkyl groups, hydroxy groups, $C_1$–$C_6$ alkoxy groups, halogen atoms, sulfo groups ($-SO_3H$), amino groups, $C_1$–$C_6$ alkylamino groups, di-$C_1$–$C_6$-alkylamino groups, $C_1$–$C_6$ alkylcarbonyl groups, $C_3$–$C_7$ cycloalkylcarbonyl groups, carb-$C_1$–$C_6$-alkoxy groups or benzoyl groups, X is oxygen, sulfur, SO or $SO_2$, Alk is $C_0$–$C_4$ alkylene and n and m may be the same or different and may assume values of 1 to 3, although n may also be 0 where Alk is alkylene containing at least one carbon atom, in which case m may assume a value of 2 to 6, and the group

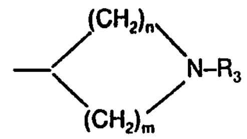

may also represent the quinuclidylradical or the tropanyl radical, and pyridine oxide thereof, characterized in that a compound corresponding to the following general formula

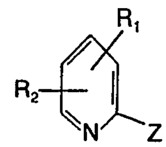
II

in which $R_1$ and $R_2$ are as defined above, or the pyridine-N-oxide thereof is reacted with a compound corresponding to the following general formula

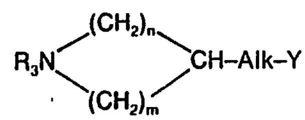
III

in which $R_3$, n and m and Alk are as already defined, in addition to which $R_3$ may also be the group S and S is a typical protective amino group and Y is a halogen atom, a $C_1$–$C_6$ alkylsulfonyloxy group or an aryl sulfonyloxy group where Z in formula II is a hydroxy group or a mercapto group or in which Y is a hydroxy group or a mercapto group where Z in formula II is a halogen atom, or an exisiting group S is eliminated and/or the substituents $R_1$, $R_2$ and $R_3$ in compounds of formula I are converted into other possible meanings and/or compounds of formula I, in which $R_1$ to $R_3$, Alk, X, n and m are as defined, are converted into the corresponding sulfones, sulfoxides or pyridine-N-oxides.

5. A process as claimed in claim 2, characterized in that the compounds obtained are converted into their salts.

6. A medicament, characterized in that it contains as active principle a compound of the type claimed in one or more of the preceding claims together with a standard pharmaceutical excipient and/or a diluent.

7. A process for the preparation of a medicament, characterized in that a compound of the type claimed in one or more of the preceding claims is processed with standard pharmaceutical excipients or diluents to form pharmaceutical preparations.

8. The use of the compounds claimed in one or more of the preceding claims for the preparation of a medicament.

## Revendications

1. Composés de formule

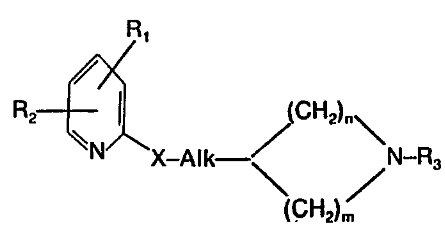
I

dans laquelle les radicaux $R_1$ et $R_2$ sont identiques ou différents et représentent chacun un atome d'hydrogène, d'halogène, un groupement trifluorométhyle, un groupement cyano, un groupement nitro, un groupement amino, un groupement mono-$C_1$–$C_6$-alkylamino, un groupement di-$C_1$–$C_6$-alkylamino, un groupement amino qui est substitué par un radical phényle, un radical mono- ou dihalogénophényle ou un radical phényl-$C_1$–$C_4$-alkyle, un groupement $C_1$–$C_6$-alcanoylamino, un groupement $C_1$–$C_6$-alcoxycarbonylamino, un groupement $C_1$–$C_6$-alkyle éventuellement substitué par un radical phényle, un groupement phényle, un groupement hydroxy, un groupement $C_1$–$C_6$-alcoxy, un groupement phénoxy, un groupement carboxy, un groupement carb-$C_1$–$C_6$-alcoxy ou un groupement carbamoyle éventuellement substitué par un ou deux groupes alkyle en $C_1$ à $C_6$, le radical $R_3$ est un atome d'hydrogène, un groupement $C_1$–$C_6$-alkyle, un groupement $C_3$–$C_6$-alcényle, un groupement $C_3$–$C_6$-alcynyle, un groupement $C_3$–$C_7$-cycloalkyle, un groupement $C_5$–$C_7$-cycloalcényle, un groupement phényl-$C_1$–$C_4$-alkyle, un groupement carb-$C_1$–$C_6$-alcoxy, un groupement $C_2$–$C_6$-alcanoyle éventuellement substitué par un radical $C_3$–$C_6$-cycloalkyle, ou un groupement $C_1$–$C_4$-alkyle qui contient, sur le même atome de carbone, deux groupements $C_1$–$C_6$-alcoxy ou un groupement $C_2$–$C_4$-alkylènedioxy, ou dans laquelle $R_3$ représente un groupement $C_1$–$C_6$-alkyle qui est substitué une fois ou deux fois par des groupements $C_3$–$C_7$-cycloalkyle, des groupements hydroxy, des groupements $C_1$–$C_6$-alcoxy, des atomes d'halogène, des groupements sulfo ($-SO_3H$), des groupements amino, des groupements $C_1$–$C_6$-alkylamino, des groupements di-$C_1$–$C_6$-alkylamino, des groupements $C_1$–$C_6$-alkylcarbonyle, des groupements $C_3$–$C_7$-cycloalkylcarbonyle, des groupements carb-$C_1$–$C_6$-alcoxy ou des groupements benzoyle, X représente un atome d'oxygène, de soufre, un radical SO ou $SO_2$, Alk est un groupement alkylène à 0–4 atomes de carbone, et n et m sont des nombres identiques ou différents et peuvent prendre des valeurs de 1 à 3, n pouvant être aussi égal à 0 lorsque Alk représente un groupement alkylène contenant au moins un atome de carbone, m prenant dans ce cas une valeur de 2 à 6, et le groupement

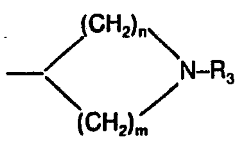

pouvant aussi représenter le radical quinuclidyle ou le radical tropanyle, ainsi que les pyridine-N-oxydes et les sels pharmaceutiquement utilisables de ces composés, à l'exception des composés 4-(2-méthyl-6-pyridyloxyméthyl)-pipéridine et 4-(2-pyridyloxyméthyl)-pipéridine.

2. Composés selon la revendication 1, dans la formule desquels $R_1$ représente un atome d'hydrogène, un groupement amino ou $C_2$–$C_6$-alcanoylamino, $R_2$ un atome de chlore, de brome, de fluor, un groupement $C_1$–$C_4$-alkyle ou $C_1$–$C_4$-alcoxy, X représente un atome de soufre et $R_3$ représente un atome d'hydrogène, un groupement $C_3$–$C_6$-alcényle ou $C_1$–$C_6$-alkyle qui peut également contenir un atome d'halogène, un groupement méthylènedioxy ou un ou deux groupements hydroxy, et dans la formule desquels le noyau saturé basique

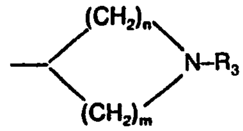

représente un radical pyrrolidyle, un radical pipéridyle ou un radical homopipéridyle qui contient le radical $R_3$ sur l'atome d'azote, ainsi que leurs sels.

3. Composés selon la revendication 1, dans la formule desquels $R_1$ représente un atome d'hydrogène, $R_2$ représente un atome de chlore, de brome ou de fluor, X représente un atome de soufre et $R_3$ représente un atome d'hydrogéne ou un groupement $C_1$–$C_6$-alkyle, et le noyau basique

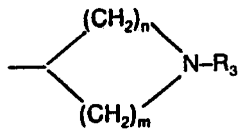

représente un radical pyrrolidyle, un radical pipéridyle ou un radical homopipéridyle qui contient le radical $R_3$ sur l'atome d'azote, ainsi que leurs sels.

4. Procédé de préparation de composés selon la revendication 1 dans lesquels les radicaux $R_1$ et $R_2$ sont identiques ou différents et représentent chacun un atome d'hydrogène, d'halogène, un groupement trifluorométhyle, un groupement cyano, un groupement nitro, un groupement amino, un groupement mono-$C_1$–$C_6$-alkylamino, un groupement di-$C_1$–$C_6$-alkylamino, un groupement amino qui est substitué par un radical phényle, un radical mono- ou dihalogénophényle ou un radical phényl-$C_1$–$C_4$-alkyle, un groupement $C_1$–$C_6$-alcanoylamino, un groupement $C_1$–$C_6$-alcoxycarbonylamino, un groupement $C_1$–$C_6$-alkyle éventuellement substitué par un radical phényle, un groupement phényle, un groupement hydroxy, un groupement $C_1$–$C_6$-alcoxy, un groupement phénoxy, un groupement carboxy, un groupement carb-$C_1$–$C_6$-alcoxy ou un groupement carbamoyle éventuellement substitué par un ou deux groupes alkyle en $C_1$ à $C_6$, le radical $R_3$ est un atome d'hydrogène, un groupement $C_1$–$C_6$-alkyle, un groupement $C_3$–$C_6$-alcényle, un groupement $C_3$–$C_6$-alcynyle, un groupement $C_3$–$C_7$-cycloalkyle, un groupement $C_5$–$C_7$-cycloalcényle, un groupement phényl-$C_1$–$C_4$-alkyle, un groupement carb-$C_1$–$C_6$-alcoxy, un groupement $C_2$–$C_6$-alcanoyle éventuellement substitué par un radical $C_3$–$C_6$-cycloalkyle, ou un groupement $C_1$–$C_4$-alkyle qui contient, sur le même atome de carbone, deux groupements $C_1$–$C_6$-alcoxy ou un groupement $C_2$–$C_4$-alkylènedioxy, ou dans lesquels $R_3$ représente un groupement $C_1$–$C_6$-alkyle qui est substitué une fois ou deux fois par des groupements $C_3$–$C_7$-cycloalkyle, des groupements hydroxy, des groupements $C_1$–$C_6$-alcoxy, des atomes d'halogène, des groupements sulfo ($-SO_3H$), des groupements amino,

des groupements $C_1$–$C_6$-alkylamino, des groupements di-$C_1$–$C_6$-alkylamino, des groupements $C_1$–$C_6$-alkylcarbonyle, des groupements $C_3$–$C_7$-cycloalkylcarbonyle, des groupements carb-$C_1$–$C_6$-alcoxy ou des groupements benzoyle, X représente un atome d'oxygène, de soufre, un radical SO ou $SO_2$, Alk est un groupement alkylène à 0–4 atomes de carbone, et n et m sont des nombres identiques ou différents et peuvent prendre des valeurs de 1 à 3, n pouvant être aussi égal à 0 lorsque Alk représente un groupement alkylène contenant au moins un atome de carbone, m prenant dans ce cas une valeur de 2 à 6, et le groupement

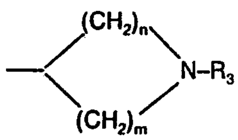

pouvant aussi représenter le radical quinuclidyle ou le radical tropanyle, ainsi que les pyridine-N-oxydes de ces composés, caractérisé en ce qu'on fait réagir un composé de formule générale

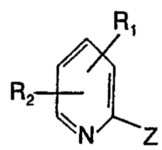

II

ou le pyridine-N-oxyde de celui-ci, dans lequel R, et $R_2$ ont les significations données ci-dessus, avec un composé de formule générale

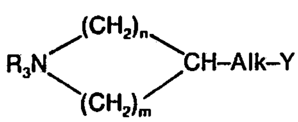

III

$R_3$, n, m et Alk dans la formule III ayant les significations données ci-dessus, $R_3$ pouvant être en outre le groupement S (S étant un groupe protecteur du groupement amino usuel) et Y étant un atome d'halogène, un groupement $C_1$–$C_6$-alkylsulfonyloxy ou un groupement arylsulfonyloxy dans le cas où Z dans la formule II est un groupement hydroxy ou un groupement mercapto, ou Y représentant un groupement hydroxy ou un groupement mercapto dans le cas où Z dans la formule II est un atome d'halogène, et on élimine un groupement S présent et/ou on transforme, dans des composés de formule I, les radicaux $R_1$, $R_2$ et $R_3$ en d'autres radicaux ayant des significations possibles pour eux et/ou transforme des composés de formule I, dans lesquels les radicaux $R_1$–$R_3$, ainsi que Alk, X, n et m ont les significations données ci-dessus, en les sulfones, les sulfoxydes ou les pyridine-N-oxydes correspondants.

5. Procédé selon la revendication 4, caractérisé en ce qu'on transforme les composés obtenus en leurs sels.

6. Médicament, caractérisé en ce qu'il contient, en tant que substance active, un composé selon l'une quelconque des revendications 1 à 5, en combinaison avec un excipient pharmaceutique usuel et/ou un diluant.

7. Procédé de préparation d'un médicament, caractérisé en ce qu'un composé selon l'une quelconque des revendications 1 à 3 est travaillé avec des excipients ou diluants pharmaceutiques usuels pour former des préparations pharmaceutiques.

8. Utilisation de composés selon l'une quelconque des revendications 1 à 3 pour la préparation d'un médicament.